# EUROPEAN PATENT APPLICATION

(11) **EP 4 471 056 A1**
(43) Date of publication of application: **04.12.2024**
(21) Application number: 23746323.7
(22) Date of filing: 20.01.2023
(51) Int. Cl.: C07K 16/18, C12N 15/13, A61K 39/395, A61P 35/00

(54) **ANTI-DKK1 ANTIBODY, PHARMACEUTICAL COMPOSITION THEREOF AND USE THEREOF**

(30) Priority: 28.01.2022 CN 202210108050
(71) Applicant: Shanghai Junshi Biosciences Co., Ltd., Pilot Free Trade Zone Shanghai 201210 (CN)
(72) Inventor: HE, Zhijuan, Shanghai 201210 (CN); WANG, Yahong, Shanghai 201210 (CN); LI, Shengsheng, Shanghai 201210 (CN); YANG, Lingzhi, Shanghai 201210 (CN); ZHOU, Yuehua, Shanghai 201210 (CN); ZHAO, Qiang, Shanghai 201210 (CN); YAO, Sheng, Shanghai 201210 (CN); FENG, Hui, Shanghai 201210 (CN)
(74) Representative: Zanoli, Enrico
(86) International application number: PCT/CN2023/073335
(87) International publication number: WO 2023/143444

(57) **Abstract**

The present invention belongs to the field of biopharmaceuticals and relates to an anti-DKK1 antibody, a pharmaceutical composition thereof, and use thereof. Specifically, the present invention relates to an anti-DKK1 antibody or an antigen-binding fragment thereof, wherein the anti-DKK1 antibody comprises a heavy chain variable region comprising HCDR1 to HCDR3 and a light chain variable region comprising LCDR1 to LCDR3, wherein the amino acid sequence of HCDR1 is selected from SEQ ID NO: 1, etc., the amino acid sequence of HCDR2 is selected from SEQ ID NO: 2, etc., the amino acid sequence of HCDR3 is selected from SEQ ID NO: 3, etc., the amino acid sequence of LCDR1 is selected from SEQ ID NO: 4, etc., the amino acid sequence of LCDR2 is selected from SEQ ID NO: 5, etc., and the amino acid sequence of LCDR3 is selected from SEQ ID NO: 6, etc. The anti-DKK1 antibody of the present invention has a good anti-tumor effect as it can bind to DKK1 with high specificity, effectively blocks the Wnt signaling pathway, and thereby inhibits the promoting effect of DKK1 on the formation of a tumor microenvironment and the promoting effect of DKK1 on the development and progression of tumors.

## Description

### TECHNICAL FIELD

The present invention belongs to the field of biopharmaceuticals and relates to an anti-DKK1 antibody, a pharmaceutical composition thereof, and use thereof.

### BACKGROUND

DKK1 is a secretory inhibitor of the Wnt/β-catenin signal transduction pathway, belongs to the DKK (dickkopf) family, one of the protein families for inhibiting Wnt signal transduction, and has the ability to inhibit Wnt-induced axial duplication. The DKK family currently has 4 family members, namely DKK1, DKK2, DKK3, and DKK4.

The Wnt signaling pathway is involved in the control of embryonic development and the tumorigenic process. Extracellular Wnt proteins are responsible for the growth and differentiation of a variety of cell types during embryonic development and contribute to the progression of a variety of cancers. Genetic analysis indicates that DKK1 inhibits Wnt signal transduction upstream, and antagonistic interaction between DKK1 and LRP6 blocks the activation of Wnt-mediated signal.

When DKK1 protein is absent, Wnt and Frizzled, LRP6 are combined to form a heterotrimer, which activates the downstream signaling pathway, maintains the stability of β-catenin, and after entering the nucleus, the heterotrimer is aggregated, and promotes the transcription and translation of an osteoblast-related gene and a cancer suppressor gene. And when DKK1 protein is present, DKK1 competes with Wnt for binding to LRP6 molecules, and a series of downstream signal reactions drive β -catenin to be phosphorylated and then degraded. Thereby this blocks β-catenin from entering the nucleus and further blocks the transcription and translation of an osteoblast-related gene and a cancer suppressor gene. When an anti-DKK1 antibody is present, the antibody competes with LRP6 for binding to DKK1 molecules, facilitating the reformation of Wnt and Frizzled, LRP6 as a heterotrimer. The downstream signaling pathway is recovered, such that the transcription and translation of the osteoblast-related gene and the cancer suppressor gene are recovered to be normal, and the effects of promoting the stability and proliferation of an osteoblasts and inhibiting the generation and proliferation of a cancer cell are achieved. DKK1 can promote the formation of a tumor microenvironment by increasing the inhibitory activity of MDSC and Treg cells, down-regulating the ligand of tumor cell surface activated NK cells, promoting the polarization of Th2 cells, and reducing the generation of IFNγ. In addition, DKK1 also promotes the tumor growth and migration, generation of CSC-like cells and angiogenesis, thereby directly promoting tumor development and progression (Yu et al., Signal Transduct Target Therapy, 2021, 6(1); Nikolai et al., Cancer Letters, 2020, 482). On March 22, 2021, LeapTherapeutics announced that their DKN-01 monoclonal antibody, which targets the target of DKK1, had positive results in the clinical treatment of advanced gynecological cancers at stage 2. Clinical results showed that DKN-01 was more effective in patients with high expression of DKK1, with an objective remission rate of 14% and a disease control rate of 57%.

DKK1 is an important regulatory factor of tumors and deserves more attention as a key therapeutic target; and in fact, strategies to develop DKK1 inhibitors have produced encouraging clinical outcomes in different pathology models (Hewen Jiang et al., Drug Discovery of DKK1 Inhibitors, Frontiers in Pharmacology, March 2022, Volume 13, p1-17). However, to date, antibody drugs directed against this target have not been marketed, with two molecules that have made the fastest progress in cancer therapy being in the clinical stage of single or combination drug studies (Jiang et al., Front Pharmacol, 2022, 13), DKN-01 (CN110114087A) in studies for multiple myeloma, gallbladder carcinoma, esophageal cancer, liver cancer, hepatocellular carcinoma, bladder cancer, prostate cancer, lung cancer, gynecological cancer, colon cancer, and rectal cancer, respectively, and BHQ-880 (WO2007084344) in studies for multiple myeloma and osteosarcoma. However, there is an unmet need to provide other more effective, specific, safe, and/or stable pharmaceutical agents that, alone or in combination with other pharmaceutical agents, can promote the expression of Wnt pathway-mediated tumor suppressor genes by enhancing the inhibitory activity against human DKK1.

### SUMMARY

The present inventors have made intensive studies and creative work to prepare a hybridoma cell line expressing an anti-DDK1 antibody and acquire the expressed anti-DDK1 antibody. Further, the present inventors designed a chimeric antibody based on this and carried out humanization. The present inventors surprisingly found that the anti-DDK1 antibody of the present invention has higher affinity and/or specificity to DDK1, can effectively inhibit tumor growth, and has good anti-tumor potential. Thus the following invention is provided:
One aspect of the present invention relates to an anti-DKK1 antibody or an antigen-binding fragment thereof,
wherein the anti-DKK1 antibody comprises a heavy chain variable region comprising HCDR1 to HCDR3 and a light chain variable region comprising LCDR1 to LCDR3,
wherein,
the amino acid sequence of HCDR1 is selected from SEQ ID NO: 1, SEQ ID NO: 7, SEQ ID NO: 13, SEQ ID NO: 19, SEQ ID NO: 25, and SEQ ID NO: 31;
the amino acid sequence of HCDR2 is selected from SEQ ID NO: 2, SEQ ID NO: 8, SEQ ID NO: 14, SEQ ID NO: 20, SEQ ID NO: 26, and SEQ ID NO: 32;
the amino acid sequence of HCDR3 is selected from SEQ ID NO: 3, SEQ ID NO: 9, SEQ ID NO: 15, SEQ ID NO: 21, SEQ ID NO: 27, SEQ ID NO: 33, and SEQ ID NO: 78;
the amino acid sequence of LCDR1 is selected from SEQ ID NO: 4, SEQ ID NO: 10, SEQ ID NO: 16, SEQ ID NO: 22, SEQ ID NO: 28, and SEQ ID NO: 34;
the amino acid sequence of LCDR2 is selected from SEQ ID NO: 5, SEQ ID NO: 11, SEQ ID NO: 17, SEQ ID NO: 23, SEQ ID NO: 29, and SEQ ID NO: 35;
the amino acid sequence of LCDR3 is selected from SEQ ID NO: 6, SEQ ID NO: 12, SEQ ID NO: 18, SEQ ID NO: 24, SEQ ID NO: 30, and SEQ ID NO: 36.

In some embodiments of the present invention, the anti-DKK1 antibody or the antigen-binding fragment thereof is provided, wherein:
for the heavy chain variable region of the antibody,
the amino acid sequence of HCDR1 is set forth in SEQ ID NO: 1, the amino acid sequence of HCDR2 is set forth in SEQ ID NO: 2, and the amino acid sequence of HCDR3 is set forth in SEQ ID NO: 3,
the amino acid sequence of HCDR1 is set forth in SEQ ID NO: 1, the amino acid sequence of HCDR2 is set forth in SEQ ID NO: 2, and the amino acid sequence of HCDR3 is set forth in SEQ ID NO: 78,
the amino acid sequence of HCDR1 is set forth in SEQ ID NO: 7, the amino acid sequence of HCDR2 is set forth in SEQ ID NO: 8, and the amino acid sequence of HCDR3 is set forth in SEQ ID NO: 9,
the amino acid sequence of HCDR1 is set forth in SEQ ID NO: 13, the amino acid sequence of HCDR2 is set forth in SEQ ID NO: 14, and the amino acid sequence of HCDR3 is set forth in SEQ ID NO: 15,
the amino acid sequence of HCDR1 is set forth in SEQ ID NO: 19, the amino acid sequence of HCDR2 is set forth in SEQ ID NO: 20, and the amino acid sequence of HCDR3 is set forth in SEQ ID NO: 21,
the amino acid sequence of HCDR1 is set forth in SEQ ID NO: 25, the amino acid sequence of HCDR2 is set forth in SEQ ID NO: 26, and the amino acid sequence of HCDR3 is set forth in SEQ ID NO: 27, or
the amino acid sequence of HCDR1 is set forth in SEQ ID NO: 31, the amino acid sequence of HCDR2 is set forth in SEQ ID NO: 32, and the amino acid sequence of HCDR3 is set forth in SEQ ID NO: 33;
   and
for the light chain variable region of the antibody,
the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6,
the amino acid sequence of LCDR1 is set forth in SEQ ID NO: 10, the amino acid sequence of LCDR2 is set forth in SEQ ID NO: 11, and the amino acid sequence of LCDR3 is set forth in SEQ ID NO: 12,
the amino acid sequence of LCDR1 is set forth in SEQ ID NO: 16, the amino acid sequence of LCDR2 is set forth in SEQ ID NO: 17, and the amino acid sequence of LCDR3 is set forth in SEQ ID NO: 18,
the amino acid sequence of LCDR1 is set forth in SEQ ID NO: 22, the amino acid sequence of LCDR2 is set forth in SEQ ID NO: 23, and the amino acid sequence of LCDR3 is set forth in SEQ ID NO: 24,
the amino acid sequence of LCDR1 is set forth in SEQ ID NO: 28, the amino acid sequence of LCDR2 is set forth in SEQ ID NO: 29, and the amino acid sequence of LCDR3 is set forth in SEQ ID NO: 30, or
the amino acid sequence of LCDR1 is set forth in SEQ ID NO: 34, the amino acid sequence of LCDR2 is set forth in SEQ ID NO: 35, and the amino acid sequence of LCDR3 is set forth in SEQ ID NO: 36.

In some embodiments of the present invention, the anti-DKK1 antibody or the antigen-binding fragment thereof is provided, wherein,
the amino acid sequence of HCDR1 is set forth in SEQ ID NO: 1, the amino acid sequence of HCDR2 is set forth in SEQ ID NO: 2, the amino acid sequence of HCDR3 is set forth in SEQ ID NO: 3, the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6; the amino acid sequence of HCDR1 is set forth in SEQ ID NO: 1, the amino acid sequence of HCDR2 is set forth in SEQ ID NO: 2, the amino acid sequence of HCDR3 is set forth in SEQ ID NO: 78, the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6; the amino acid sequence of HCDR1 is set forth in SEQ ID NO: 7, the amino acid sequence of HCDR2 is set forth in SEQ ID NO: 8, the amino acid sequence of HCDR3 is set forth in SEQ ID NO: 9, the amino acid sequence of LCDR1 is set forth in SEQ ID NO: 10, the amino acid sequence of LCDR2 is set forth in SEQ ID NO: 11, and the amino acid sequence of LCDR3 is set forth in SEQ ID NO: 12;
the amino acid sequence of HCDR1 is set forth in SEQ ID NO: 13, the amino acid sequence of HCDR2 is set forth in SEQ ID NO: 14, the amino acid sequence of HCDR3 is set forth in SEQ ID NO: 15, the amino acid sequence of LCDR1 is set forth in SEQ ID NO: 16, the amino acid sequence of LCDR2 is set forth in SEQ ID NO: 17, and the amino acid sequence of LCDR3 is set forth in SEQ ID NO: 18;
the amino acid sequence of HCDR1 is set forth in SEQ ID NO: 19, the amino acid sequence of HCDR2 is set forth in SEQ ID NO: 20, the amino acid sequence of HCDR3 is set forth in SEQ ID NO: 21, the amino acid sequence of LCDR1 is set forth in SEQ ID NO: 22, the amino acid sequence of LCDR2 is set forth in SEQ ID NO: 23, and the amino acid sequence of LCDR3 is set forth in SEQ ID NO: 24;
the amino acid sequence of HCDR1 is set forth in SEQ ID NO: 25, the amino acid sequence of HCDR2 is set forth in SEQ ID NO: 26, the amino acid sequence of HCDR3 is set forth in SEQ ID NO: 27, the amino acid sequence of LCDR1 is set forth in SEQ ID NO: 28, the amino acid sequence of LCDR2 is set forth in SEQ ID NO: 29, and the amino acid sequence of LCDR3 is set forth in SEQ ID NO: 30;
   or
the amino acid sequence of HCDR1 is set forth in SEQ ID NO: 31, the amino acid sequence of HCDR2 is set forth in SEQ ID NO: 32, the amino acid sequence of HCDR3 is set forth in SEQ ID NO: 33, the amino acid sequence of LCDR1 is set forth in SEQ ID NO: 34, the amino acid sequence of LCDR2 is set forth in SEQ ID NO: 35, and the amino acid sequence of LCDR3 is set forth in SEQ ID NO: 36.

Antigen binding is dependent on the variable regions of the light chain and the heavy chain; the variable region of each chain contains three hypervariable regions, called complementarity determining regions (CDRs) (CDRs of the heavy chain (H) comprise HCDR1, HCDR2, and HCDR3, and CDRs of the light chain (L) comprise LCDR1, LCDR2, and LCDR3, named by Kabat et al., see Bethesda M.d., Sequences of Proteins of Immunological Interest, Fifth Edition, NIH Publication 1991; 1-3:91-3242).

In some embodiments of the present invention, the anti-DKK1 antibody or the antigen-binding fragment thereof is provided, wherein,
the amino acid sequence of the heavy chain variable region of the antibody is selected from SEQ ID NO: 37, SEQ ID NO: 39, SEQ ID NO: 41, SEQ ID NO: 43, SEQ ID NO: 45, SEQ ID NO: 47, SEQ ID NOs: 49-53, SEQ ID NOs: 58-62, and SEQ ID NOs: 68-73; and
the amino acid sequence of the light chain variable region of the antibody is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs:74-77.

In some embodiments of the present invention, the anti-DKK1 antibody or the antigen-binding fragment thereof is provided, wherein,
the amino acid sequence of the heavy chain variable region of the antibody is set forth in SEQ ID NO: 37, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs:54-57, SEQ ID NOs: 63-67, and SEQ ID NOs: 74-77;
the amino acid sequence of the heavy chain variable region of the antibody is set forth in SEQ ID NO: 39, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 41, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 43, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 45, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 47, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 49, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 50, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 51, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 52, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 53, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 58, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 59, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 60, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 61, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 62, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 68, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 69, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 70, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 71, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 72, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
   or
the amino acid sequence of the heavy chain variable region of the antibody is set forth in SEQ ID NO: 73, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77.

In some embodiments of the present invention, the anti-DKK1 antibody or the antigen-binding fragment thereof is provided, wherein,
the amino acid sequence of a heavy chain of the antibody is selected from SEQ ID NOs: 79-83, SEQ ID NOs: 88-92, and SEQ ID NOs: 98-103; and
the amino acid sequence of a light chain of the antibody is selected from SEQ ID NOs: 84-87, SEQ ID NOs: 93-97, and SEQ ID NOs: 104-107.

In some embodiments of the present invention, the anti-DKK1 antibody or the antigen-binding fragment thereof is provided, wherein,
the amino acid sequence of the heavy chain of the antibody is set forth in SEQ ID NO: 79, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 80, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 81, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 82, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 83, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 88, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 89, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 90, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 91, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 92, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 98, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 99, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 100, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 101, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 102, and the amino acid sequence of the light chain is selected from SEQ ID NOs: 84-87, SEQ ID NOs: 93-97, and SEQ ID NOs: 104-107;
   or
the amino acid sequence of the heavy chain of the antibody is set forth in SEQ ID NO: 103, and the amino acid sequence of the light chain is selected from SEQ ID NOs: 84-87, SEQ ID NOs: 93-97, and SEQ ID NOs: 104-107.

In some embodiments of the present invention, for the anti-DKK1 antibody or the antigen-binding fragment thereof, wherein the anti-DKK1 antibody or the antigen-binding fragment thereof is selected from a Fab, a Fab', an F(ab')2, an Fd, an Fv, a dAb, a complementarity determining region fragment, a single-chain antibody, a humanized antibody, a chimeric antibody, or a diabody.

Another aspect of the present invention relates to an anti-DKK1 antibody or an antigen-binding fragment thereof having the following characteristics:
(a) binding to an epitope of a human DKK1 protein that is identical to, completely overlaps with, or partially overlaps with an epitope to which the antibody or the antigen-binding fragment described in any one of the embodiments herein binds; or
(b) competing for binding to an epitope of a human DKK1 protein with the antibody or the antigen-binding fragment described in any one of the embodiments herein.

In some embodiments of the present invention, the anti-DKK1 antibody or the antigen-binding fragment thereof is provided, wherein,
the antibody comprises a non-CDR region, and the non-CDR region is from a species except for murine, such as from a human antibody.

In some embodiments of the present invention, the anti-DKK1 antibody or the antigen-binding fragment thereof is provided, wherein a heavy chain constant region of the antibody is Ig gamma-1 chain C region (e.g., NCBI ACCESSION: P01857) or Ig gamma-4 chain C region (e.g., NCBI ACCESSION: P01861.1), and a light chain constant region is Ig kappa chain C region (e.g., NCBI ACCESSION: P01834);
preferably, the amino acid sequence of the heavy chain constant region of the antibody is set forth in SEQ ID NO: 112, and the amino acid sequence of the light chain constant region of the antibody is set forth in SEQ ID NO: 113.

Another aspect of the present invention relates to an isolated nucleic acid molecule encoding the anti-DKK1 antibody or the antigen-binding fragment thereof described in any one of the embodiments herein.

A further another aspect of the present invention relates to a recombinant vector comprising the isolated nucleic acid molecule of the present invention.

A further another aspect of the present invention relates to a host cell comprising the isolated nucleic acid molecule of the present invention or the recombinant vector of the present invention.

A further another aspect of the present invention relates to a conjugate comprising an antibody and a conjugated moiety, wherein the antibody is the anti-DKK1 antibody or the antigen-binding fragment thereof described in any one of the embodiments herein, and the conjugated moiety is a detectable label; preferably, the conjugated moiety is a cytotoxin, an immunoregulator, a radioisotope, a fluorescent substance, a luminescent substance, a colored substance, or an enzyme.

A further another aspect of the present invention relates to a kit comprising the anti-DKK1 antibody or the antigen-binding fragment thereof described in any one of the embodiments herein or the conjugate of the present invention;
preferably, the kit further comprises a second antibody that specifically recognizes the antibody; optionally, the second antibody further comprises a detectable label, such as a cytotoxin, an immunoregulator, a radioisotope, a fluorescent substance, a luminescent substance, a colored substance, or an enzyme.

A further another aspect of the present invention relates to a bispecific antibody comprising a first functional protein domain and a second functional protein domain, wherein:
the first functional protein domain targets DKK1, and
the second functional protein domain targets a target different from DKK1, wherein the first functional protein domain is the antibody or the antigen-binding fragment described in any one of the embodiments herein.

In some embodiments of the present invention, the bispecific antibody is provided, wherein the first functional protein domain and the second functional protein domain are linked directly or via a linker moiety.

In some embodiments of the present invention, the bispecific antibody is provided, wherein the number of the first functional protein domain and that of the second functional protein domain are independently 1, 2, or more than 2.

In some embodiments of the present invention, the bispecific antibody is provided, wherein the single-chain antibodies are linked to the C-termini of two heavy chains of an immunoglobulin-form antibody, respectively.

A further another aspect of the present invention relates to a pharmaceutical composition comprising the anti-DKK1 antibody or the antigen-binding fragment thereof described in any one of the embodiments herein or the conjugate of the present invention;
optionally, the pharmaceutical composition further comprises one or more other anti-tumor agents; preferably, the other anti-tumor agent is a chemotherapeutic agent or an antibody; preferably, the chemotherapeutic agent is a tubulin inhibitor; preferably, the antibody drug is an immune checkpoint inhibitor; preferably, the chemotherapeutic agent is paclitaxel;
optionally, the pharmaceutical composition further comprises one or more pharmaceutically acceptable auxiliary materials (e.g., carriers and/or excipients).

In one or more embodiments of the present invention, the pharmaceutical composition is provided, wherein a mass ratio of the anti-DKK1 antibody or the antigen-binding fragment thereof to paclitaxel is (1:5)-(5:1), such as 1:5, 1:4, 1:3, 1:2, 1:1, 2:1, 3:1, 4:1, or 5:1, based on the mass of the antibody.

A further another aspect of the present invention relates to a combination product comprising a first product and a second product in separate packages, wherein, the first product comprises the anti-DKK1 antibody or the antigen-binding fragment thereof described in any one of the embodiments herein or the conjugate of the present invention;
the second product comprises one or more other anti-tumor agents; preferably, the other anti-tumor agent is a chemotherapeutic agent or an antibody; preferably, the chemotherapeutic agent is a tubulin inhibitor; preferably, the antibody drug is an immune checkpoint inhibitor; preferably, the chemotherapeutic agent is paclitaxel;
preferably, the first product and the second product further independently comprise one or more pharmaceutically acceptable auxiliary materials (e.g., carriers and/or excipients);
preferably, the combination product further comprises a product instruction.

In one or more embodiments of the present invention, the combination product is provided, wherein a mass ratio of the anti-DKK1 antibody or the antigen-binding fragment thereof to paclitaxel is (1:5)-(5:1), such as 1:5, 1:4, 1:3, 1:2, 1:1, 2:1, 3:1, 4:1, or 5:1, based on the mass of the antibody.

A further another aspect of the present invention relates to use of the antibody or the antigen-binding fragment thereof described in any one of the embodiments herein, the conjugate of the present invention, the bispecific antibody described in any one of the embodiments herein, or the pharmaceutical composition described in any one of the embodiments herein in preparing a medicament for treating and/or preventing a DKK1-mediated disease. Preferably, the DKK1-mediated disease is a tumor; preferably, the tumor is selected from one or more of gallbladder carcinoma, bladder cancer, osteosarcoma, esophageal cancer, cholangio carcinoma, endometrial cancer, ovarian cancer, gastric cancer, gastroesophageal junction cancer, colon cancer, rectal cancer, hepatocellular carcinoma, prostate cancer, lung cancer, and myeloma.

Provided is the antibody or the antigen-binding fragment thereof described in any one of the embodiments herein, the conjugate of the present invention, the bispecific antibody described in any one of the embodiments herein, or the pharmaceutical composition described in any one of the embodiments herein for use in treating and/or preventing a DKK1-mediated disease. Preferably, the DKK1-mediated disease is a tumor; preferably, the tumor is selected from one or more of gallbladder carcinoma, bladder cancer, osteosarcoma, esophageal cancer, cholangio carcinoma, endometrial cancer, ovarian cancer, gastric cancer, gastroesophageal junction cancer, colon cancer, rectal cancer, hepatocellular carcinoma, prostate cancer, lung cancer, and myeloma.

A further another aspect of the present invention relates to a method for treating or preventing a DKK1-mediated disease comprising the step of administering to a subject in need thereof an effective amount of the antibody or the antigen-binding fragment thereof described in any one of the embodiments herein, the conjugate of the present invention, the bispecific antibody described in any one of the embodiments herein, or the pharmaceutical composition described in any one of the embodiments herein. Preferably, the DKK1-mediated disease is a tumor; preferably, the tumor is selected from one or more of gallbladder carcinoma, bladder cancer, osteosarcoma, esophageal cancer, cholangio carcinoma, endometrial cancer, ovarian cancer, gastric cancer, gastroesophageal junction cancer, colon cancer, rectal cancer, hepatocellular carcinoma, prostate cancer, lung cancer, and myeloma.

Preferably, the route of administration is intravenous drip infusion or intravenous injection.

In some embodiments of the present invention, the method for treating or preventing a DKK1-mediated disease is a method for treating or preventing a tumor. In some embodiments of the present invention, the method for treating or preventing a tumor further comprises the step of co-administering to the subject one or more therapies comprising surgical treatment and/or radiation therapy and/or administering one or more other anti-tumor agents;
preferably, the other anti-tumor agent is a chemotherapeutic agent or an antibody; preferably, the chemotherapeutic agent is a tubulin inhibitor; preferably, the antibody drug is an immune checkpoint inhibitor; preferably, the chemotherapeutic agent is paclitaxel.

### BRIEF DESCRIPTION OF THE DRAWINGS

FIG. 1: Assay on hybridoma antibodies' blocking of the binding of DDK1 to LRP6 by ELISA.
FIG. 2A: Assay on hybridoma antibodies' antagonistic effect of the inhibition effect of DDK1 to Wnt signal by luciferase reporter gene method.
FIG. 2B: Assay on hybridoma antibodies' antagonistic effect of the inhibition effect of DDK1 to Wnt signal by luciferase reporter gene method.
FIG. 3: Assay on chimeric antibodies' blocking of the binding of DDK1 to LRP6 by ELISA.
FIG. 4A: Assay on the binding of chimeric antibodies to DKK1 by ELISA.
FIG. 4B: Assay on the binding of chimeric antibodies to DKK1 by ELISA.
FIG. 5A: Assay on chimeric antibodies' effect in recovering the Wnt signaling pathway by luciferase reporter gene method.
FIG. 5B: Assay on chimeric antibodies' effect in recovering the Wnt signaling pathway by luciferase reporter gene method.
FIG. 5C: Assay on chimeric antibodies' effect in recovering the Wnt signaling pathway by luciferase reporter gene method.
FIG. 6: Assay on the binding of humanized antibodies to DDK1 by ELISA.
FIG. 7: Assay on humanized antibodies' blocking of the binding of DDK1 to LRP6 by ELISA.
FIG. 8: Assay on humanized antibodies' antagonistic effect of the inhibition effect of DKK1 to Wnt signal by luciferase reporter gene method.
FIG. 9: Inhibition of tumor growth by humanized antibody hu3.
FIG. 10: Inhibition of tumor growth by humanized antibody hu3.

The partial sequences (the underlined parts represent the CDRs) to which the present invention relates
SEQ ID NO: 1
   EYTMH
SEQ ID NO: 2
   VIDPSNGDTSYNQKFKG
SEQ ID NO: 3
   TTGPWFAS
SEQ ID NO: 4
   RASQDISNYLN
SEQ ID NO: 5
   YKSRLHS
SEQ ID NO: 6
   QQVHTLPPT
SEQ ID NO: 7
   ENTMH
SEQ ID NO: 8
   GINPHKGGTSYNQKFKG
SEQ ID NO: 9
   DWLGAMDY
SEQ ID NO: 10
   ITSTDIDDDMN
SEQ ID NO: 11
   EGNTLRP
SEQ ID NO: 12
   LQSDNLPYT
SEQ ID NO: 13
   SYNML
SEQ ID NO: 14
   VIFPGNGDTSYNPKFKG
SEQ ID NO: 15
   DGYPSMDY
SEQ ID NO: 16
   ITSTDIDDDMN
SEQ ID NO: 17
   EGNSLRP
SEQ ID NO: 18
   LQFNNMPLT
SEQ ID NO: 19
   TSGMGVG
SEQ ID NO: 20
   HIWWDDVKRYNPALKS
SEQ ID NO: 21
   IARSPTGFAY
SEQ ID NO: 22
   SASSSVSYMY
SEQ ID NO: 23
   DTSNLAS
SEQ ID NO: 24
   QQWSSYPPALT
SEQ ID NO: 25
   AFDVH
SEQ ID NO: 26
   VIWIGGTTDYNGAFIS
SEQ ID NO: 27
   KRGNYYNMDY
SEQ ID NO: 28
   RSSTGTVTTTNYAN
SEQ ID NO: 29
   GTNNRAP
SEQ ID NO: 30
   ALWYSNHWV
SEQ ID NO: 31
   NHGMN
SEQ ID NO: 32
   WINTNTGEPTYADDFKG
SEQ ID NO: 33
   RGYGFPYYYTMDY
SEQ ID NO: 34
   RSSTGAVTTRNYAN
SEQ ID NO: 35
   GTKNRAP
SEQ ID NO: 36
   ALWYSNRLV
SEQ ID NO: 37 
MGWSWIFLFLLSGTAGVLSEVQLQQSGPELVKPGASVRISCKTSGYTFTE
YTMHWVKQSHGKSLEWIGVIDPSNGDTSYNQKFKGRATLTVDKSSSTA
YMDLRSLTSEDSALYYCSSTTGPWFACWGQGTLVTVSA
SEQ ID NO: 38 
MSSAQFLGLLLLCFQGTRCDIQMTQTTSSLSASLGDRVTINCRASQDISN
YLNWYQQKPDGTVKLLIYYKSRLHSGVPTRFSGSGSGTDFSLTISNLVQE
DIATYFCQQVHTLPPTFGGGTKLEIK
SEQ ID NO: 39 
MGWSWIFLFLLSGTAGVLSEVQLQQSGPELVKPGASVKIFCKTSGYTFTE
NTMHWVKQTHGKSLEWIGGINPHKGGTSYNQKFKGKATLTVDKSSSTA
CMELRSLTSEDSTVYYCARDWLGAMDYWGQGTSVTVSP
SEQ ID NO: 40 
MFSLALLLSLLLLCVSDSRAETTVTQSPASLSMAIGEKVTIRCITSTDIDD
DMNWYQQKPGEPPKLLISEGNTLRPGVPSRFSSSGYGTDFVFTIENMLSE
DVADYYCLQSDNLPYTFGGGTKLEIK
SEQ ID NO: 41 
MGWNCFILFLVATATGVHSQVQLQQPGAELVKPGASVKMSCKASGYTFT
SYNMLWVKQTPGQGLEWIGVIFPGNGDTSYNPKFKGKAALTADKSSSTA
YMQLSSLTSEDSAVYYCARDGYPSMDYWGQGTSVTVSS
SEQ ID NO: 42 
MLSLAPLLSLLLLCVSDSRAETIVTQSPASLSVATGEKVTIRCITSTDIDDD
MNWYQQKPGEPPKLLISEGNSLRPGVPSRFSSSGYGTDFVFTIENTLSED
VADYYCLQFNNMPLTFGAGTKLELK
SEQ ID NO: 43 
MGRLTSSFLLLIVPAYVLSQVTLKESGPGILQPSQTLSLTCSFSGFSLSTSG
MGVGWIRQPSGKGLEWLAHIWWDDVKRYNPALKSRLTISKDTSSSQVF
LKIASVDTTDTASYYCSRIARSPTGFAYWGQGTL
SEQ ID NO: 44 
MDFQVQIFSFLLISASVILSRGQIVLTQSPVIMSASPGEKVTMTCSASSSVS
YMYWYQQKPGSSPRLLIYDTSNLASGVPVRFSGSGSGTSYSLTISRMEAE
DAATYYCQQWSSYPPALTFGAGTKLELK
SEQ ID NO: 45 
MAVLGLLFCLVTFPSCVLSQVQLKQSGPGLVQPSQSLSITCTVSGFSLTAF
DVHWVRQSPGKGLEWLGVIWIGGTTDYNGAFISRLSISKDNSKSQVFFK
MNSLQAKDTAIYYCARKRGNYYNMDYWGQGTSVTVAS
SEQ ID NO: 46 
MAWISLILSLLALSSGAISQAVVTQESALTTSPGETVTLTCRSSTGTVTTTN
YANWVQEKPDHLFTGLIGGTNNRAPGVPARFSGSLIGDKAALTITGAQTE
DEAIYFCALWYSNHWVFGGGTKLTVL
SEQ ID NO: 47 
MAWVWTLLFLMAAAQSAQAQIQLVQSGPELKKPGETVKISCKASGYTF
TNHGMNWVKQAPGKGLKWMGWINTNTGEPTYADDFKGRFAFSLETSA
STAYLQINNLKNEDTATYFCARRGYGFPYYYTMDYWGQGTSVTVSS
SEQ ID NO: 48 
MAWISLILSLLALSSGAISQAVVTQESALTTSPGETVTLTCRSSTGAVTTR
NYANWVQEKADHLLTGLIGGTKNRAPGVPARFSGSLIGDKAALTITGAQ
TEDEAKYFCALWYSNRLVFGGGTKLTVL
SEQ ID NO: 49 
EVQLLQSAAEVKRPGESLRISCKTSGYTFTEYTMHWVRQMPGKELEWM
GVIDPSNGDTSYNQKFKGHVTISADKSSSTAYLQWSSLKASDAAMYYCS
STTGPWFASWGQGTLVTVSS
SEQ ID NO: 50 
QVQLVQSGAEVKKPGASVKVSCKASGYTFTEYTMHWVRQAPGQGLEW
MGVIDPSNGDTSYNQKFKGRVTLTVDKSTSTAYMELRSLRSDDTAVYYC
ARTTGPWFASWGQGTLVTVSS
SEQ ID NO: 51 
QVQLVQSGAEVKKPGSSVKVSCKASGYTFTEYTMHWVRQAPGQGLEW
MGVIDPSNGDTSYNQKFKGRVTITADKSTSTAYMELSSLRSEDTAVYYCS
STTGPWFASWGQGTLVTVSS
SEQ ID NO: 52 
QVQLVQSGAEVKKPGASVKVSCKASGYTFTEYTMHWVRQAPGQRLEW
MGVIDPSNGDTSYNQKFKGRVTITVDKSSSTAYMELSSLRSEDTAVYYCS
STTGPWFASWGQGTLVTVSS
SEQ ID NO: 53 
QVQLVQSGAEVKKPGASVKVSCKASGYTFTEYTMHWVRQAPGQRLEW
MGVIDPSNGDTSYNQKFKGRVTLTVDKSSSTAYMELSSLRSEDTAVYYC
 ARTTGPWFASWGQGTLVTVSS
SEQ ID NO: 54 
DIQMTQSPSSLSASVGDRVTITCRASQDISNYLNWYQQKPGKAPKLLIYY
KSRLHSGVPSRFSGSGSGTDFTFTISSLQPEDIATYYCQQVHTLPPTFGQG
TKLEIK
SEQ ID NO: 55 
DIQMTQSPSSLSASVGDRVTITCRASQDISNYLNWYQQKPGKAPKLLIYY
KSRLHSGVPSRFSGSGSGTDFTLTISSLQPEDFATYYCQQVHTLPPTFGQG
TKLEIK
SEQ ID NO: 56 
EIVMTQSPPTLSLSPGERVTLSCRASQDISNYLNWYQQKPGQAPRLLIYY
KSRLHSGIPARFSGSGSGTDFTLTISSLQPEDFAVYYCQQVHTLPPTFGQG
TKLEIK
SEQ ID NO: 57 
DIQMTQSPSSLSASVGDRVTITCRASQDISNYLNWYQQKPGKAPKLLIYY
KSRLHSGVPSRFSGSGSGTDFTFTISSLQPEDIATYFCQQVHTLPPTFGQG
TKLEIK
SEQ ID NO: 58 
EVQLLQSAAEVKRPGESLRISCKTSGYSFTENTMHWVRQMPGKELEWM
GGINPHKGGTSYNQKFKGHVTISVDKSSSTAYLQWSSLKASDAAMYYC
ARDWLGAMDYWGQGTLVTVSS
SEQ ID NO: 59 EVQLLQSAAEVKRPGESLRISCKTSGYTFTENTMHWVRQMPGKELEWM 
GGINPHKGGTSYNQKFKGHVTISADKSSSTAYLQWSSLKASDAAMYYC
ARDWLGAMDYWGQGTLVTVSS
SEQ ID NO: 60 
EVQLLQSAAEVKRPGESLRISCKTSGYTFTENTMHWVRQMPGKELEWM
GGINPHKGGTSYNQKFKGHVTLSVDKSSSTAYLQWSSLKASDAAMYYC
ARDWLGAMDYWGQGTLVTVSS
SEQ ID NO: 61 
QVQLVQSGAEVKKPGASVKVSCKASGYTFTENTMHWVRQAPGQGLEW
MGGINPHKGGTSYNQKFKGRVTLTVDKSSSTAYMELSRLRSDDTAVYYC
ARDWLGAMDYWGQGTLVTVSS
SEQ ID NO: 62 
QVQLVQSGAEVKKPGSSVKVSCKASGYTFTENTMHWVRQAPGQGLEW
MGGINPHKGGTSYNQKFKGRVTITVDKSTSTAYMELSSLRSEDTAVYYC
ARDWLGAMDYWGQGTLVTVSS
SEQ ID NO: 63 
DIQLTQSPSSLSASVGDRVTITCITSTDIDDDMNWYRQKPGKVPKLLISEG
NTLRPGVPSRFSGSGSGTDFTLTISSLQPEDVATYYCLQSDNLPYTFGQGT
KLEIK
SEQ ID NO: 64 
DIQVTQSPSSLSASVGDRVTITCITSTDIDDDMNWYQQKPGKAPKLLISE
GNTLRPGVPSRFSGSGSGTDFTFTISSLQPEDIATYYCLQSDNLPYTFGQG
TKLEIK
SEQ ID NO: 65 
DIVLTQSPASLAVSPGQRATITCITSTDIDDDMNWYQQKPGQPPKLLISEG
NTLRPGVPARFSSSGSGTDFTLTINPVEANDTANYYCLQSDNLPYTFGQG
TKLEIK
SEQ ID NO: 66 
DTVLTQSPASLAVSPGQRATITCITSTDIDDDMNWYQQKPGQPPKLLISEG
NTLRPGVPARFSSSGSGTDFTLTINPVEANDTANYYCLQSDNLPYTFGQG
TKLEIK
SEQ ID NO: 67 
DIVLTQSPASLAVSPGQRATITCITSTDIDDDMNWYQQKPGQPPKLLISEG
NTLRPGVPARFSSSGSGTDFTLTINPVEANDVANYYCLQSDNLPYTFGQG
TKLEIK
SEQ ID NO: 68 
QVQLVQSGAEVKKPGASVKVSCKASGYTFTSYNMLWVRQAPGQRLEW
MGVIFPGNGDTSYNPKFKGRVTITADKSSSTAYMELSSLRSEDTAVYYCA
RDGYPSMDYWGQGTLVTVSS
SEQ ID NO: 69 
QVQLVQSGAEVKKPGASVKVSCKASGYTFTSYNMLWVRQAPGQRLEW
MGVIFPGNGDTSYNPKFKGRVTLTADKSSSTAYMELSSLRSEDTAVYYCA
RDGYPSMDYWGQGTLVTVSS
SEQ ID NO: 70 
QVQLVQSGAEVKKPGSSVKVSCKASGYTFSSYNMLWVRQAPGQGLEW
MGVIFPGNGDTSYNPKFKGRVTITADKSTSTAYMELSSLRSEDTAVYYCA
RDGYPSMDYWGQGTLVTVSS
SEQ ID NO: 71 
QVQLVQSGAEVKKPGSSVKVSCKASGYTFTSYNMLWVRQAPGQGLEW
MGVIFPGNGDTSYNPKFKGRVTITADKSTSTAYMELSSLRSEDTAVYYCA
RDGYPSMDYWGQGTLVTVSS
SEQ ID NO: 72 
QVQLVQSGAEVKKPGSSVKVSCKASGYTFTSYNMLWVRQAPGQGLEW
MGVIFPGNGDTSYNPKFKGRVTLTADKSSSTAYMELSSLRSEDTAVYYCA
RDGYPSMDYWGQGTLVTVSS
SEQ ID NO: 73 
QVQLVQSGAEVKKPGASVKVSCKASGYTFTSYNMLWVRQAPGQRLEW
MGVIFPGNADTSYNPKFKGRVTLTADKSSSTAYMELSSLRSEDTAVYYCA
RDGYPSMDYWGQGTLVTVSS
SEQ ID NO: 74 
DIQMTQSPSSVSASVGDRVTITCITSTDIDDDMNWYQQKPGKAPKLLISE
GNSLRPGVPSRFSGSGSGTDFTLTISSLQPEDFATYYCLQFNNMPLTFGQG
TKLEIK
SEQ ID NO: 75 
DIQLTQSPSFLSASVGDRVTITCITSTDIDDDMNWYQQKPGKAPKLLISEG
NSLRPGVPSRFSSSGSGTEFTLTISSLQPEDFATYYCLQFNNMPLTFGQGT
KLEIK
SEQ ID NO: 76 
DTQLTQSPSFLSASVGDRVTITCITSTDIDDDMNWYQQKPGKAPKLLISE
GNSLRPGVPSRFSSSGSGTEFTLTISSLQPEDFATYYCLQFNNMPLTFGQG
TKLEIK
SEQ ID NO: 77 
EIVLTQSPDFQSVTPKEKVTITCITSTDIDDDMNWYQQKPDQSPKLLISEG
NSLRPGVPSRFSSSGSGTDFTLTINSLEAEDAATYYCLQFNNMPLTFGQGT
KLEIK
SEQ ID NO: 78
   TTGPWFAC
SEQ ID NO: 79 
EVQLLQSAAEVKRPGESLRISCKTSGYTFTEYTMHWVRQMPGKELEWM
GVIDPSNGDTSYNQKFKGHVTISADKSSSTAYLQWSSLKASDAAMYYCS
STTGPWFASWGQGTLVTVSSASTKGPSVFPLAPCSRSTSESTAALGCLVK
DYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSL
GK
SEQ ID NO: 80 
QVQLVQSGAEVKKPGASVKVSCKASGYTFTEYTMHWVRQAPGQGLEW
MGVIDPSNGDTSYNQKFKGRVTLTVDKSTSTAYMELRSLRSDDTAVYYC
ARTTGPWFASWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
 
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 81 
QVQLVQSGAEVKKPGSSVKVSCKASGYTFTEYTMHWVRQAPGQGLEW
MGVIDPSNGDTSYNQKFKGRVTITADKSTSTAYMELSSLRSEDTAVYYCS
STTGPWFASWGQGTLVTVSSASTKGPSVFPLAPCSRSTSESTAALGCLVK
DYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSL
GK
SEQ ID NO: 82 
QVQLVQSGAEVKKPGASVKVSCKASGYTFTEYTMHWVRQAPGQRLEW
MGVIDPSNGDTSYNQKFKGRVTITVDKSSSTAYMELSSLRSEDTAVYYCS
STTGPWFASWGQGTLVTVSSASTKGPSVFPLAPCSRSTSESTAALGCLVK
DYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSL
GK
SEQ ID NO: 83 
QVQLVQSGAEVKKPGASVKVSCKASGYTFTEYTMHWVRQAPGQRLEW
MGVIDPSNGDTSYNQKFKGRVTLTVDKSSSTAYMELSSLRSEDTAVYYC
ARTTGPWFASWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 84 
DIQMTQSPSSLSASVGDRVTITCRASQDISNYLNWYQQKPGKAPKLLIYY
KSRLHSGVPSRFSGSGSGTDFTFTISSLQPEDIATYYCQQVHTLPPTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 85 
DIQMTQSPSSLSASVGDRVTITCRASQDISNYLNWYQQKPGKAPKLLIYY
KSRLHSGVPSRFSGSGSGTDFTLTISSLQPEDFATYYCQQVHTLPPTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 86 
EIVMTQSPPTLSLSPGERVTLSCRASQDISNYLNWYQQKPGQAPRLLIYY
KSRLHSGIPARFSGSGSGTDFTLTISSLQPEDFAVYYCQQVHTLPPTFGQG
TKLEIKRTVAAPSVFIFPPSDEQLKSGTASVVCLLNNFYPREAKVQWKVD
 
NALQSGNSQESVTEQDSKDSTYSLSSTLTLSKADYEKHKVYACEVTHQG
LSSPVTKSFNRGEC
SEQ ID NO: 87 
DIQMTQSPSSLSASVGDRVTITCRASQDISNYLNWYQQKPGKAPKLLIYY
KSRLHSGVPSRFSGSGSGTDFTFTISSLQPEDIATYFCQQVHTLPPTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 88 
EVQLLQSAAEVKRPGESLRISCKTSGYSFTENTMHWVRQMPGKELEWM
GGINPHKGGTSYNQKFKGHVTISVDKSSSTAYLQWSSLKASDAAMYYC
ARDWLGAMDYWGQGTLVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLGK
SEQ ID NO: 89 
EVQLLQSAAEVKRPGESLRISCKTSGYTFTENTMHWVRQMPGKELEWM
GGINPHKGGTSYNQKFKGHVTISADKSSSTAYLQWSSLKASDAAMYYC
ARDWLGAMDYWGQGTLVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQF
 
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLGK
SEQ ID NO: 90 
EVQLLQSAAEVKRPGESLRISCKTSGYTFTENTMHWVRQMPGKELEWM
GGINPHKGGTSYNQKFKGHVTLSVDKSSSTAYLQWSSLKASDAAMYYC
ARDWLGAMDYWGQGTLVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLGK
SEQ ID NO: 91 
QVQLVQSGAEVKKPGASVKVSCKASGYTFTENTMHWVRQAPGQGLEW
MGGINPHKGGTSYNQKFKGRVTLTVDKSSSTAYMELSRLRSDDTAVYYC
ARDWLGAMDYWGQGTLVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLGK
SEQ ID NO: 92 
QVQLVQSGAEVKKPGSSVKVSCKASGYTFTENTMHWVRQAPGQGLEW
MGGINPHKGGTSYNQKFKGRVTITVDKSTSTAYMELSSLRSEDTAVYYC
ARDWLGAMDYWGQGTLVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLGK
SEQ ID NO: 93 
DIQLTQSPSSLSASVGDRVTITCITSTDIDDDMNWYRQKPGKVPKLLISEG
NTLRPGVPSRFSGSGSGTDFTLTISSLQPEDVATYYCLQSDNLPYTFGQGT
KLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGL
SSPVTKSFNRGEC
SEQ ID NO: 94 
DIQVTQSPSSLSASVGDRVTITCITSTDIDDDMNWYQQKPGKAPKLLISE
GNTLRPGVPSRFSGSGSGTDFTFTISSLQPEDIATYYCLQSDNLPYTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 95 
DIVLTQSPASLAVSPGQRATITCITSTDIDDDMNWYQQKPGQPPKLLISEG
NTLRPGVPARFSSSGSGTDFTLTINPVEANDTANYYCLQSDNLPYTFGQG
 
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 96 
DTVLTQSPASLAVSPGQRATITCITSTDIDDDMNWYQQKPGQPPKLLISEG
NTLRPGVPARFSSSGSGTDFTLTINPVEANDTANYYCLQSDNLPYTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 97 
DIVLTQSPASLAVSPGQRATITCITSTDIDDDMNWYQQKPGQPPKLLISEG
NTLRPGVPARFSSSGSGTDFTLTINPVEANDVANYYCLQSDNLPYTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 98 
QVQLVQSGAEVKKPGASVKVSCKASGYTFTSYNMLWVRQAPGQRLEW
MGVIFPGNGDTSYNPKFKGRVTITADKSSSTAYMELSSLRSEDTAVYYCA
RDGYPSMDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 99 
QVQLVQSGAEVKKPGASVKVSCKASGYTFTSYNMLWVRQAPGQRLEW
MGVIFPGNGDTSYNPKFKGRVTLTADKSSSTAYMELSSLRSEDTAVYYCA
RDGYPSMDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 100 
QVQLVQSGAEVKKPGSSVKVSCKASGYTFSSYNMLWVRQAPGQGLEW
MGVIFPGNGDTSYNPKFKGRVTITADKSTSTAYMELSSLRSEDTAVYYCA
RDGYPSMDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 101 
QVQLVQSGAEVKKPGSSVKVSCKASGYTFTSYNMLWVRQAPGQGLEW
MGVIFPGNGDTSYNPKFKGRVTITADKSTSTAYMELSSLRSEDTAVYYCA
RDGYPSMDYWGQGTLVTVSSASTKGPSVFPLAPCSRSTSESTAALGCLV
 
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 102 
QVQLVQSGAEVKKPGSSVKVSCKASGYTFTSYNMLWVRQAPGQGLEW
MGVIFPGNGDTSYNPKFKGRVTLTADKSSSTAYMELSSLRSEDTAVYYCA
RDGYPSMDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLS
LGK
SEQ ID NO: 103 
QVQLVQSGAEVKKPGASVKVSCKASGYTFTSYNMLWVRQAPGQRLEW
MGVIFPGNADTSYNPKFKGRVTLTADKSSSTAYMELSSLRSEDTAVYYCA
RDGYPSMDYWGQGTLVTVSSASTKGPSVFPLAPCSRSTSESTAALGCLV
KDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTP
 
PVLDSDGSFFLYSRLTVDKSRWQEGNVFSCSVMHEALHNHYTQKSLSLS
LGK
SEQ ID NO: 104 
DIQMTQSPSSVSASVGDRVTITCITSTDIDDDMNWYQQKPGKAPKLLISE
GNSLRPGVPSRFSGSGSGTDFTLTISSLQPEDFATYYCLQFNNMPLTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 105 
DIQLTQSPSFLSASVGDRVTITCITSTDIDDDMNWYQQKPGKAPKLLISEG
NSLRPGVPSRFSSSGSGTEFTLTISSLQPEDFATYYCLQFNNMPLTFGQGT
KLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGL
SSPVTKSFNRGEC
SEQ ID NO: 106 
DTQLTQSPSFLSASVGDRVTITCITSTDIDDDMNWYQQKPGKAPKLLISE
GNSLRPGVPSRFSSSGSGTEFTLTISSLQPEDFATYYCLQFNNMPLTFGQG
TKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 107 
EIVLTQSPDFQSVTPKEKVTITCITSTDIDDDMNWYQQKPDQSPKLLISEG
NSLRPGVPSRFSSSGSGTDFTLTINSLEAEDAATYYCLQFNNMPLTFGQGT
KLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGL
 SSPVTKSFNRGEC
SEQ ID NO: 108 
EVQLVESGGGLVQPGGSLRLSCAASGFTFSSYTMSWVRQAPGKGLEWV
ATISGGGFGTYYPDSVKGRFTISRDNAKNSLYLQMNSLRAEDTAVYYCA
RPGYNNYYFDIWGQGTTVTVSSASTKGPSVFPLAPCSRSTSESTAALGCL
VKDYFPEPVTVSWNSGALTSGVHTFPAVLQSSGLYSLSSVVTVPSSSLGT
KTYTCNVDHKPSNTKVDKRVESKYGPPCPPCPAPEAAGGPSVFLFPPKPK
DTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSL
SLG
SEQ ID NO: 109 
EIVLTQSPATLSLSPGERATLSCHASDSISNSLHWYQQKPGQAPRLLIYYA
RQSIQGIPARFSGSGSGTDFTLTISSLEPEDFAVYYCQQSESWPLHFGGGT
KVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQG
LSSPVTKSFNRGEC
SEQ ID NO: 110 
QVQLVESGGGLVQPGGSLRLSCAASGFTFSSYWMSWVRQAPGKGLEW
VSGISYSGSNTHYADSVKGRFTISRDNSKNTLYLQMNSLRAEDTAVYYC
ARMGIDLDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQ
TYICNVNHKPSNTKVDKRVEPKSCDKTHTCPPCPAPELLGGPSVFLFPPK
PKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQP
 
REPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSL
SLSPGK
SEQ ID NO: 111 
DIALTQPASVSGSPGQSITISCTGTSSDVGGFNYVSWYQQHPGKAPKLMI
HDGSNRPSGVSNRFSGSKSGNTASLTISGLQAEDEADYYCQSWDVSPITA
VFGGGTKLTVLGQPKAAPSVTLFPPSSEELQANKATLVCLISDFYPGAVT
VAWKADSSPVKAGVETTTPSKQSNNKYAASSYLSLTPEQWKSHRSYSCQ
VTHEGSTVEKTVAPTECS
SEQ ID NO: 112 
ASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVES
KYGPPCPPCPAPEFLGGPSVFLFPPKPKDTLMISRTPEVTCVVVDVSQEDP
EVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCL
VKGFYPSDIAVEWESNGQPENNYKTTPPVLDSDGSFFLYSRLTVDKSRW
QEGNVFSCSVMHEALHNHYTQKSLSLSLGK
SEQ ID NO: 113

### DETAILED DESCRIPTION

The present invention provides an anti-DKK1 antibody or an antigen-binding fragment thereof characterized by having a unique CDR sequence composition and being capable of binding to DKK1 with high affinity and high specificity. The anti-DKK1 antibody or the antigen-binding fragment thereof provided herein can be used as an independent therapy or in combination with other therapies and/or other anti-cancer pharmaceutical agents to treat, such as, cancers.

### Definitions

Unless otherwise stated, embodiments of the present invention will employ conventional techniques of molecular biology (including recombinant techniques), microbiology, cytobiology, biochemistry, and immunology, which are all within the skill of the art.

In order to facilitate the understanding of the present invention, some technical and scientific terms are specifically defined as follows. Unless otherwise specifically defined herein, all technical and scientific terms used herein have the same meaning as commonly understood by those of ordinary skill in the art to which the present invention belongs. For definitions and terminology in the art, those skilled can refer specifically to Current Protocolsin Molecular Biology (Ausubel). Abbreviations for amino acid residues are standard 3-letter and/or 1-letter codes used in the art to denote one of the 20 commonly used L-amino acids. The singular forms used herein (including claims) include their corresponding plural forms, unless otherwise explicitly specified herein.

The term "about" used in combination with a numerical value is intended to encompass the numerical values in a range from a lower limit less than the specified numerical value by 5% to an upper limit greater than the specified numerical value by 5%.

The term "DKK1" herein, a secretory inhibitor of the Wnt/β-catenin signal transduction pathway, belonging to the DKK (dickkopf) family (one of the protein families for inhibiting Wnt signal transduction), refers to any native DKK1 from any vertebrate animal (including mammals, such as primates (e.g., human) and rodents (e.g., mice and rats)), unless otherwise indicated. This term encompasses "full-length" unprocessed DKK1 and DKK1 in any form resulting from intracellular processing or any fragment thereof. The term also includes variants of naturally occurring DKK1, such as splice variants or allelic variants. In some embodiments, DKK1 refers to full-length DKK1 from humans and mice or fragments thereof (such as mature fragments thereof lacking a signal peptide). In some embodiments, DKK1 refers to a mature human DKK1 identical to a sequence of amino acid residues 32-266 (Genbank accession No. NP_036374.1) (SEQ ID NO: 31) (amino acid residues 1-31 are leader peptides).

The "percent (%) amino acid sequence identity" is defined as the percentage of amino acid residues in a candidate sequence that are identical to those in a reference polypeptide sequence after aligning the sequences (with gaps introduced if necessary) to achieve maximum percent sequence identity without considering any conservative substitution as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring alignment, including any algorithm required to obtain maximum alignment for the full length of the aligned sequences.

The term "immune response" refers to the action of, such as, lymphocytes, antigen-presenting cells, phagocytes, granulocytes, and soluble macromolecules produced by the above cells or liver (including antibodies, cytokines and complements) that results in selective damage to, destruction of, or elimination from the human body of invading pathogens, cells or tissues infected with pathogens, cancerous cells, or, in the cases of autoimmunity or pathological inflammation, normal human cells or tissues.

The term "signal transduction pathway" or "signal transduction activity" refers to a biochemical causal relationship generally initiated by a protein-protein interaction (such as binding of a growth factor to a receptor) and resulting in transmission of a signal from one portion of a cell to another portion of the cell. In general, the transmission includes specific phosphorylation of one or more tyrosine, serine, or threonine residues on one or more proteins in a series of reactions causing signal transduction. The penultimate process typically involves a nuclear event, resulting in a change in gene expression.

The term "activity" or "bioactivity", or the term "biological property" or "biological characteristic" can be used interchangeably herein and includes, but is not limited to, epitope/antigen affinity and specificity, the ability to neutralize or antagonize the activity of DKK1 *in vivo* or *in vitro,* IC50, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain a high expression level of the protein in mammalian cells. The aforementioned properties or characteristics are observed, determined, or assessed using techniques well known in the art, including but not limited to ELISA, FACS, or BIACORE plasma resonance analysis, unlimited *in vitro* or *in vivo* neutralization assays, receptor binding, cytokine or growth factor production and/or secretion, signal transduction, and immunohistochemistry of tissue sections of different sources (including human, primate or any other source). An "antibody" refers to any form of antibody having a desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

An "isolated antibody" refers to the purified state of a binding compound, and, in this case, means that the molecule is substantially free of other biomolecules, such as nucleic acids, proteins, lipids, sugars, or other substances such as cell debris and growth medium. The term "isolate(d)" does not mean the complete absence of such substances or the absence of water, buffers, or salts, unless they are present in amounts that will significantly interfere with the experimental or therapeutic use of the binding compounds described herein.

A "monoclonal antibody" refers to an antibody obtained from a substantially homogeneous population of antibodies, i.e., the antibodies comprising the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method. A "full-length antibody" refers to an immunoglobulin molecule comprising four peptide chains when present naturally, including two heavy (H) chains (about 50-70 kDa in full length) and two light (L) chains (about 25 kDa in full length) linked to each other by disulfide bonds. Each of the heavy chains consists of a heavy chain variable region (abbreviated herein as VH) and a heavy chain constant region (abbreviated herein as CH). The heavy chain constant region consists of 3 domains, i.e., CH1, CH2, and CH3. Each of the light chains consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, and FR4. The variable regions of the heavy and light chains contain binding domains that interact with antigens. The constant regions of an antibody can mediate the binding of immunoglobulins to host tissues or factors, including various cells of the immune system (e.g., effector cells) and the first component (C1q) of classical complement system.

An "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of the binding fragment of an antibody include, but are not limited to, Fab, Fab', F(ab')2, and Fv fragments; a diabody; a linear antibody; a single-chain antibody molecule, such as sc-Fv; a nanobody and a multispecific antibody formed by fragments of the antibody. The binding fragment or the derivative generally retains at least 10% of the antigen-binding activity of the parent antibody when the antigen-binding activity is expressed on a molar concentration basis. Preferably, the binding fragment or the derivative retains at least 20%, 50%, 70%, 80%, 90%, 95%, or 100% or more of the antigen binding affinity of the parent antibody. It is also contemplated that the antigen-binding fragment of the antibody may include conservative or non-conservative amino acid substitutions that do not significantly alter its bioactivity (referred to as "conservative variants" or "function-conservative variants" of the antibody). The term "binding compound" refers to both the antibody and the binding fragment thereof.

A "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

A "domain antibody" is an immunofunctional immunoglobulin fragment that contains only the heavy chain variable region or the light chain variable region. In certain cases, two or more VH regions are covalently linked to a peptide linker to form a bivalent domain antibody. The two VH regions of the bivalent domain antibody may target the same or different antigens.

A "bivalent antibody" comprises two antigen-binding sites. In certain cases, the two binding sites have the same antigen specificity. However, the bivalent antibody may be bispecific.

A "diabody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of the other chain to form two antigen-binding sites.

A "chimeric antibody" is an antibody having the variable domains of a first antibody and the constant domains of a second antibody, wherein the first and second antibodies are from different species. Typically, the variable domain is obtained from an antibody of an experimental animal such as a rodent ("parent antibody"), and the constant domain sequence is obtained from a human antibody, such that the resulting chimeric antibody is less likely to induce an adverse immune response in a human subject as compared to the parent rodent antibody. A "humanized antibody" refers to an antibody form containing sequences from both human and non-human (such as mouse and rat) antibodies. In general, the humanized antibody comprises substantially all of at least one, and typically two, variable domains, in which all or substantially all of the hypervariable loops are equivalent to those of a non-human immunoglobulin and all or substantially all of the framework regions (FRs) are those of a human immunoglobulin sequence. The humanized antibody may comprise optional at least a portion of a human immunoglobulin constant region (Fc).

A "fully human antibody" refers to an antibody that comprises only human immunoglobulin sequences. A fully human antibody may contain mouse glycochains if produced in mice, mouse cells, or hybridomas derived from mouse cells. Likewise, "a mouse antibody" refers to an antibody that comprises only mouse immunoglobulin sequences. Alternatively, a fully human antibody may contain rat glycochains if produced in rats, rat cells, or hybridomas derived from rat cells. Likewise, a "rat antibody" refers to an antibody that comprises only rat immunoglobulin sequences.

"Isotypes" of antibodies refer to types of antibodies (e.g., IgM, IgE, and IgG (such as IgG1, IgG2, or IgG4)) provided by heavy chain constant region genes. Isotype also includes modified forms of one of these types in which modifications have been made to alter Fc function, such as, to enhance or attenuate effector function or binding to Fc receptors.

The term "nucleic acid" or "polynucleotide" refers to deoxyribonucleic acid (DNA) or ribonucleic acid (RNA) and polymers thereof in either single- or double-stranded form. Unless explicitly limited, the term includes nucleic acids containing known analogs of natural nucleotides that have binding properties similar to that of the reference nucleic acid and are metabolized in a manner similar to naturally occurring nucleotides (see U.S. Pat. No. 8,278,036 to Kariko et al., which discloses an mRNA molecule with uridine replaced by pseudouridine, a method for synthesizing the mRNA molecule, and a method for delivering a therapeutic protein *in vivo*)*.* Unless otherwise specified, a particular nucleic acid sequence also implicitly includes conservatively modified variants thereof (e.g., degenerate codon substitutions), alleles, orthologs, SNPs, and complementary sequences as well as the sequence explicitly indicated. Specifically, degenerate codon substitutions can be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule (such as plasmid, cosmid, virus, autonomously replicating polynucleotide molecule, phage, or linear or circular single- or double-stranded DNA or RNA polynucleotide molecule) derived from any source, capable of genomic integration or autonomous replication, and comprising a polynucleotide molecule where one or more polynucleotide molecules have been linked in a functionally operative manner (i.e., operably linked). The recombinant construct typically comprises a polynucleotide of the present invention operably linked to transcription initiation regulatory sequences that will direct transcription of the polynucleotide in a host cell. Both heterologous and non-heterologous (i.e., endogenous) promoters can be used to direct the expression of the nucleic acids of the present invention.

"Vector" refers to any recombinant polynucleotide construct that can be used for transformation purpose (i.e., the introduction of heterologous DNA into a host cell). One type of vector is a "plasmid", which refers to a circular double-stranded DNA loop into which additional DNA segments can be ligated. Another type of vector is a viral vector, in which additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of the host cell upon introduction into a host cell, and are thereby replicated along with the host genome. In addition, certain vectors are capable of directing the expression of operably linked genes. Such vectors are referred to herein as "expression vectors". The term "expression vector" as used herein refers to a nucleic acid molecule capable of replicating and expressing a target gene when transformed, transfected, or transduced into a host cell. The expression vector comprises one or more phenotypic selectable markers and an origin of replication to ensure the maintenance of the vector and to provide amplification in the host if needed.

Unless otherwise or explicitly specified in the context, "activation", "stimulation" and "treatment" for a cell or a receptor may have the same meaning. For example, the cell or the receptor is activated, stimulated, or treated with a ligand. "Ligands" include natural and synthetic ligands, such as cytokines, cytokine variants, analogs, mutant proteins, and binding compounds derived from antibodies.

"Ligands" also include small molecules, such as peptidomimetics of cytokines and peptidomimetics of antibodies. "Activation" may refer to the activation of a cell regulated by internal mechanisms and external or environmental factors. "Response/reaction", e.g., a response of a cell, a tissue, an organ, or an organism, includes changes in biochemical or physiological behaviors (e.g., concentration, density, adhesion or migration, gene expression rate, or differentiation state within a biological compartment), where the changes are associated with activation, stimulation or treatment, or are associated with an internal mechanism such as genetic programming.

As used herein, the term "treat", "treating" or "treatment" of any disease or disorder refers in one embodiment to ameliorating the disease or disorder (i.e., slowing or arresting or reducing the progression of the disease or at least one of its clinical symptoms). In another embodiment, "treat", "treating" or "treatment" refers to ameliorating or alleviating at least one physical parameter, including those physical parameters that may not be discernible by the patient. In another embodiment, "treat", "treating" or "treatment" refers to modulating the disease or disorder, physically (e.g., stabilization of discernible symptoms), physiologically (e.g., stabilization of physical parameters), or both. Unless explicitly described herein, methods for assessing treatment and/or prevention of disease are generally known in the art.

A "subject" includes any human or non-human animal. The term "non-human animal" includes all vertebrates, e.g., mammals and non-mammals, such as non-human primates, sheep, dogs, cats, horses, cattle, chickens, amphibians, and reptiles.

Administration "in combination with" one or more other anti-tumor agents includes simultaneous (co-) administration and sequential administration in any order.

"Therapeutically effective amount", "therapeutically effective dose" and "effective amount" refer to an amount of the anti-DKK1 antibody or the antigen-binding fragment thereof of the present invention that is effective in preventing or ameliorating one or more symptoms of a disease or condition or the progression of the disease or condition when administered alone or in combination with other therapeutic drugs to a cell, a tissue or a subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose refers to the amount of the ingredient. In the case of administration in combination, a therapeutically effective dose refers to the combined amount of active ingredients that produce a therapeutic effect, regardless of whether these active ingredients are administered in combination, sequentially, or simultaneously. A therapeutically effective amount will increase a diagnostic index or parameter by at least 10%, generally at least 20%, preferably at least about 30%, more preferably at least 40%, and most preferably at least 50%.

"Cancer" and "cancerous" refer to or describe the physiological condition in mammals which is typically characterized by unregulated cell growth. Included in this definition are benign and malignant cancers as well as dormant tumors or micrometastases.

Anti-DKK1 Antibody and Production Thereof

The term "anti-DKK1 antibody", "anti-DKK1", "DKK1 antibody" or "DKK1-binding antibody" refers to an antibody that is capable of binding to a DKK1 protein or a fragment thereof with sufficient affinity such that the antibody can be used as a diagnostic and/or therapeutic agent targeting DKK1.

Any suitable method for producing antibodies may be employed to produce the antibody of the present invention. DKK1 in any suitable form may be used as an immunogen (antigen) for antibody production. By way of example rather than limitation, any DKK1 variant or a fragment thereof may be used as an immunogen. In some embodiments, hybridoma cells that produce murine monoclonal anti-human DKK1 antibodies can be produced by methods well known in the art. These methods include, but are not limited to, hybridoma techniques originally developed by Kohler et al., (1975) (Nature 256:495-497). Preferably, mouse splenocytes are isolated and fused to a mouse myeloma cell line using PEG or by electrofusion according to standard schemes. Hybridoma cells secreting an antibody with DKK1-inhibiting activity are then screened. The DNA sequence of the immunoglobulin variable region of the hybridoma cells of the present invention may be determined using a degenerate primer-based PCR method.

Antibodies derived from rodents (e.g., mouse) may induce unwanted immunogenicity of the antibodies when used as therapeutic agents *in vivo.* Repeated use of these antibodies induces an immune response in the human body to therapeutic antibodies. Such immune responses result in at least a loss of therapeutic efficacy and, for severe cases, a potentially lethal allergic reaction. One method for reducing the immunogenicity of rodent antibodies includes producing chimeric antibodies, in which the mouse variable region is fused to the human constant region (Liu et al., (1987) Proc. Natl. Acad. Sci. USA 84:3439-43). However, the preservation of intact rodent variable region in a chimeric antibody can still induce deleterious immunogenicity in patients. Grafting of the complementarity determining region (CDR) loops of the rodent variable domain onto the human framework (i.e., humanization) has been used to further minimize rodent sequences (Jones et al., (1986) Nature 321:522; Verhoeyen et al., (1988) Science 239:1534).

In some embodiments, the chimeric or humanized antibodies of the present invention can be prepared based on the sequences of the prepared murine monoclonal hybridoma antibodies. DNA encoding the immunoglobulin heavy and light chains can be obtained from a murine hybridoma of interest and engineered to comprise non-murine (e.g., human) immunoglobulin sequences using standard molecular biology techniques.

In some embodiments, for the chimeric DKK1 antibodies described herein, the chimeric heavy chains and the chimeric light chains can be obtained by operably linking the immunoglobulin heavy chain and light chain variable regions of hybridoma origin to human IgG constant regions respectively using methods known in the art (see, e.g., U.S. Pat. No.4,816,567 to Cabilly et al.). In some embodiments, the chimeric antibodies of the present invention comprise constant regions which can be selected from any subtype of human IgG, such as IgG1, IgG2, IgG3, and IgG4, preferably IgG4.

In some embodiments, the chimeric DKK1 antibodies of the present invention can be obtained by "mixing and matching" a chimeric light chain expression plasmid with a chimeric heavy chain expression plasmid to transfect expression cells. The DKK1 binding of such "mixed and matched" antibodies can be assayed using the above binding assays and other conventional binding assays (e.g., ELISA).

The precise amino acid sequence boundaries of the variable region CDRs of the antibodies of the present invention can be determined using any of a number of well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948

(1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures. The boundaries of the CDRs of the antibodies of the present invention can be determined by one skilled in the art according to any scheme (e.g., different assignment systems or combinations) in the art.

It should be noted that the boundaries of the CDRs of the variable regions of the same antibody obtained based on different assignment systems may differ. That is, the CDR sequences of the variable regions of the same antibody defined under different assignment systems are different. Thus, when it comes to defining an antibody with a particular CDR sequence defined in the present invention, the scope of the antibody also encompasses antibodies whose variable region sequences comprise the particular CDR sequence but whose claimed CDR boundaries differ from the particular CDR boundaries defined in the present invention due to the application of different schemes (e.g., different assignment systems or combinations).

Antibodies with different specificities (i.e., different binding sites for different antigens) have different CDRs. However, although CDRs vary from antibody to antibody, only a limited number of amino acid positions within a CDR are directly involved in antigen binding. The smallest overlapping region can be determined using at least two of the Kabat, Chothia, AbM, Contact and North methods, thereby providing a "smallest binding unit" for antigen binding. The smallest binding unit may be a sub-portion of the CDR. As will be clear to those skilled in the art, the residues in the remainder of the CDR sequences can be determined by the antibody structure and protein folding. Thus, variants of any of the CDRs presented herein are also contemplated by the present invention. For example, in a variant of one CDR, the amino acid residue of the smallest binding unit may remain unchanged, while the remaining CDR residues defined according to Kabat or Chothia may be replaced by conservative amino acid residues.

For the humanized antibodies described herein, murine CDR regions can be inserted into human germline framework regions using methods known in the art. See U.S. Pat. No. 5,225,539 to Winter et al. and U.S. Pat. Nos. 5,530,101, 5,585,089, 5,693,762 and 6,180,370 to Queen et al. Briefly, human germline IgG genes homologous to the cDNA sequence of the murine antibody variable regions were retrieved in the human immunoglobulin gene database of the NCBI (http://www.ncbi.nlm.nih.gov/igblast/) by the inventor, and in principle, humanization is achieved by grafting of the selected CDRs. However, CDR loop exchange still fails to uniformly produce an antibody with the same binding properties as the initial antibody. In humanized antibodies, changes in framework residues (FRs) (residues involved in CDR loop support) are often required to maintain the antigen-binding affinity. Briefly, the humanization comprises the following steps: A. comparing the gene sequence of each candidate antibody with the gene sequence of the human embryonic antibody to find out a sequence with high homology; B. analyzing and inspecting HLA-DR affinity, and selecting a human embryonic framework sequence with low affinity; and C. analyzing the framework amino acid sequences of the variable regions and their periphery by using a computer simulation technology and applying molecular docking to investigate their spatial and stereo combination modes. The key amino acid individuals that can interact with DKK1 and maintain the spatial framework in the gene sequence of the candidate antibodies were analyzed by calculating electrostatic force, Van der Waals' force, hydrophilicity and hydrophobicity, and entropy value, and grafted to the selected human embryonic gene framework. The amino acid sites of the framework regions which must be retained were mapped. After that, the humanized antibodies were synthesized.

In some embodiments, one or more amino acid modifications may be introduced into an Fc region of the antibody provided herein, thus producing an Fc region variant. The Fc region variant may comprise human Fc region sequences (e.g., human IgG1, IgG2, IgG3, or IgG4 Fc regions) which comprise amino acid modifications (e.g., substitutions) at one or more amino acid positions.

In some embodiments, antibodies modified by cysteine engineering may need to be produced, such as "sulfo-MAb", wherein one or more residues of the antibodies are substituted by cysteine residues.

In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

In some embodiments, the anti-DKK1 antibody or the antigen-binding fragment thereof described herein has one or more of the following properties: (1) specifically binding to human DKK1 protein; (2) cross-reacting with cynomolgus monkey DKK1; (3) inhibiting the binding of DKK1 to LRP5 or LRP6; (4) inhibiting DKK1-mediated activity signal transduction; (5) significantly inhibiting the growth of tumors.

In some embodiments, the anti-DKK1 antibody or the antigen-binding fragment thereof described herein has at least one of the following properties:
(1) binding to human DKK1 with a K_{D} of at least about 5 nM, at least about 1 nM, at least about 0.1 nM, at least about 0.01 nM, or at least about 0.001 nM;
(2) cross-reacting with cynomolgus monkey DKK1.

The present invention also relates to a method for producing the antibody or the antigen-binding fragment thereof described herein. Such a method comprises providing an isolated nucleic acid molecule encoding the antibody or the antigen-binding fragment of the present invention or an expression vector comprising such a nucleic acid molecule, particularly a vector for recombinant production of the antibody or the antigen-binding fragment thereof described herein in a host cell. The present invention also relates to a host cell comprising one or more aforementioned recombinant or expression vectors and a method for producing the antibody or the antigen-binding fragment thereof described herein, wherein the method comprises: culturing the host cell and purifying and isolating the antibody or the antigen-binding fragment thereof.

The present invention also provides a method for producing the aforementioned antibody or the antigen-binding fragment thereof, which comprises: culturing the aforementioned host cell, and isolating the antibody or the antigen-binding fragment thereof from the culture.

The present invention also relates to a method for treating cancer in a subject, which comprises: administering to the subject an effective amount of any of the anti-DKK1 antibodies or the antigen-binding fragments thereof or the pharmaceutical compositions thereof described herein.

In one embodiment, the present invention also relates to use of any of the anti-DKK1 antibodies or the antigen-binding fragments thereof described herein in preparing a medicament for treating cancer in a subject.

The present invention also provides use of the aforementioned antibody or antigen-binding fragment thereof, nucleic acid molecule, vector, host cell or pharmaceutical composition in preparing a medicament for treating and/or preventing a DKK1-mediated disease or disorder, wherein preferably, the disease or disorder is cancer.

The present invention also relates to the co-administration of one or more therapies (e.g., treatment modalities and/or other anti-tumor agents) to a subject with cancer. In some embodiments, the aforementioned therapy comprises: administering to the subject an effective amount of the anti-DKK1 antibody or the antigen-binding fragment thereof or the pharmaceutical composition thereof according to any one of the embodiments described herein. In some embodiments, the treatment modalities include surgical treatment and radiation therapy. In some embodiments, the aforementioned other anti-tumor agents comprise a chemotherapeutic agent and/or an immune checkpoint inhibitor, wherein the chemotherapeutic agent is selected from a tubulin inhibitor and/or an anti-angiogenic agent and is preferably a tubulin inhibitor.

The present invention also relates to use of a combination of any of the anti-DKK1 antibodies or the antigen-binding fragments thereof described herein and a tubulin inhibitor in preparing a medicament for treating cancer in a subject.

The present invention also relates to a method for detecting DKK1 in a sample, which comprises: a) contacting the sample with any of the anti-DKK1 antibodies or the fragments thereof described herein; and b) detecting the formation of a complex of the anti-DKK1 antibody or the fragment thereof with DKK1. In one embodiment, the anti-DKK1 antibody is detectably labeled.

In some embodiments, the present invention relates to a kit or an article of manufacture, which comprises any of the anti-DKK1 antibodies or the fragments thereof described herein. In some embodiments, the kit or the article of manufacture comprises the anti-DKK1 antibody or the fragment thereof described herein, optionally a pharmaceutically acceptable auxiliary material, and optionally one or more other anti-tumor agents (e.g., an immune checkpoint inhibitor, a tubulin inhibitor, an anti-angiogenic agent or other chemotherapeutic agents).

The present invention also encompasses any combination of the embodiments described herein. Any of the embodiments described herein or any combination thereof is applicable to any and all of the anti-DKK1 antibodies or the fragments thereof, the methods, and the uses described herein.

In some embodiments, the anti-DKK1 antibody or the antigen-binding fragment thereof described herein specifically binds to DKK1 with a KD of about l nM-2 pM or higher affinity, wherein preferably, it specifically binds to DKK1 with a KD or affinity of 1 nM, 100 pM, 10 pM, or 2 pM. In one embodiment, the antibody of the present invention that specifically binds to human DKK1 also cross-reacts with cynomolgus monkey DKK1. As used herein, "cross-reactivity" refers to the ability of an antibody to react with homologous proteins from other species. Whether an antibody specifically binds to human DKK1 may be determined using any assay method known in the art. Examples of assay methods for determining binding affinity known in the art include surface plasmon resonance (e.g., BIACORE) or similar techniques (e.g., ForteBio).

In some embodiments, the anti-DKK1 antibody or the antigen-binding fragment thereof described herein has inhibitory activity, e.g., inhibiting the expression (e.g., the expression of DKK1 on the surface of cells), activity and/or signaling of DKK1, or interfering with the interaction between DKK1 and LRP5 or LRP6. The anti-DKK1 antibody provided herein completely or partially reduces or regulates the expression or activity of DKK1 upon binding to or interacting with DKK1 (e.g., human DKK1). The biological function of DKK1 is completely, significantly or partially reduced or regulated upon the interaction between the antibody and human DKK1 polypeptide and/or peptide. The antibody described herein is believed to be capable of completely inhibiting the expression or activity of DKK1 when the level of expression or activity of DKK1 is reduced by at least 95% (e.g., 96%, 97%, 98%, 99% or 100%) in the presence of the antibody as compared to the level of expression or activity of DKK1 in the absence of interaction (e.g., binding) with the antibody. The DKK1 antibody described herein is believed to be capable of significantly inhibiting the expression or activity of DKK1 when the level of expression or activity of DKK1 is reduced by at least 50% (e.g., 55%, 60%, 75%, 80%, 85% or 90%) in the presence of the DKK1 antibody as compared to the level of expression or activity of DKK1 in the absence of binding to the DKK1 antibody. The antibody described herein is believed to be capable of partially inhibiting the expression or activity of DKK1 when the level of expression or activity of DKK1 is reduced by less than 95%

(e.g., 10%, 20%, 25%, 30%, 40%, 50%, 60%, 75%, 80%, 85% or 90%) in the presence of the antibody as compared to the level of expression or activity of DKK1 in the absence of interaction (e.g., binding) with the antibody.

### Antibody Expression

In one aspect, the present invention relates to a host cell comprising one or more expression vectors and a method for producing any of the antibodies or fragments thereof of the present invention, and the method comprises: culturing the host cell, and purifying and isolating the antibody or the antigen-binding fragment.

In one aspect, the present invention provides a nucleic acid encoding any of the aforementioned anti-DKK1 antibodies or fragments thereof. The nucleic acid can include a nucleic acid encoding an amino acid sequence of the light chain variable region and/or heavy chain variable region of the antibody, or a nucleic acid encoding an amino acid sequence of the light chain and/or heavy chain of the antibody.

In some embodiments, one or more vectors comprising the nucleic acid are provided. In one embodiment, the vector is an expression vector.

The present invention provides a mammalian host cell for expressing the recombinant antibody of the present invention, which includes a number of immortalized cell lines available from American Type Culture Collection (ATCC). Those cell lines include, in particular, Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells, and many other cell lines. Mammalian host cells include human, mouse, rat, dog, monkey, pig, goat, cow, horse, and hamster cells. Particularly preferred cell lines are selected by determining which cell line has a high expression level.

In one embodiment, the present invention provides a method for preparing an anti-DKK1 antibody, wherein the method comprises: introducing an expression vector into a mammalian host cell, and culturing the host cell for a period of time sufficient to allow expression of the antibody in the host cell or more preferably to allow secretion of the antibody into a medium in which the host cell is grown, thereby producing the antibody. The antibody can be isolated from the medium using standard protein purification methods.

It is likely that antibodies expressed by different cell lines or in transgenic animals have different glycosylations from each other. However, all antibodies encoded by the nucleic acid molecules provided herein or comprising the amino acid sequences provided herein are integral parts of the present invention, regardless of the glycosylation of the antibody. Likewise, in certain embodiments, nonfucosylated antibodies are advantageous because they generally have more potent efficacy *in vitro* and *in vivo* than their fucosylated counterparts, and are unlikely to be immunogenic because their glycan structures are normal components of natural human serum IgG.

### Pharmaceutical Composition and Pharmaceutical Formulation

The present invention provides a pharmaceutical composition comprising one or more antibodies or antigen-binding fragments thereof that bind to DKK1, and the composition comprises the antibody or the antigen-binding fragment thereof as described in any one embodiment above and a pharmaceutically acceptable carrier or excipient.

It should be understood that the anti-DKK1 antibodies or the antigen-binding fragments thereof or the pharmaceutical composition thereof provided herein can be integrated into a suitable carrier, an excipient and other reagents in a formulation for administration in combination, thus providing improved transfer, delivery, tolerance, etc.

The term "pharmaceutical composition" refers to a formulation which allows the biological activity of active ingredients contained therein to be present in an effective form and does not contain additional ingredients having toxicity unacceptable to a subject to which the formulation is administered.

The pharmaceutical formulation comprising the anti-DKK1 antibody or the antigen-binding fragment thereof described herein, preferably in the form of an aqueous solution or a lyophilized formulation, may be prepared by mixing the anti-DKK1 antibody or the antigen-binding fragment thereof of the present invention having the desired purity with one or more optional pharmaceutical auxiliary materials (Remington's Pharmaceutical Sciences, 16th edition, Osol, A. Ed. (1980)).

The pharmaceutical composition or formulation of the present invention can further comprise one or more additional active ingredients that are required for a specific indication being treated, preferably active ingredients having complementary activities that do not adversely affect one another. In some embodiments, the additional active ingredients are chemotherapeutic agents, tubulin inhibitors, immune checkpoint inhibitors, growth inhibitors, antibiotics or various known anti-tumor or anti-cancer agents, which are suitably present in combination in amounts that are effective for the purpose intended. In some embodiments, the pharmaceutical composition of the present invention also comprises a composition of a polynucleotide encoding the anti-DKK1 antibody or the antigen-binding fragment thereof.

### Medical Use of Antibodies

In one aspect, the present invention relates to a method for administering to a subject an effective amount of any of the anti-DKK1 antibodies or the antigen-binding fragments thereof described herein, an immunoconjugate comprising the antibody or the antigen-binding fragment thereof or the pharmaceutical composition for inducing T cell- or NK cell-mediated anti-tumor activity, promoting cancer inhibition activity associated with the classical Wnt pathway or enhancing the immune response of the body.

In another aspect, the present invention relates to a method for administering to a subject an effective amount of any of the anti-DKK1 antibodies or the antigen-binding fragments thereof described herein, an immunoconjugate comprising the antibody or the antigen-binding fragment or the pharmaceutical composition for treating or delaying various cancers, immune-related diseases and T cell dysfunctional diseases.

In another aspect, the present invention provides a method for treating or preventing cancer in a subject, wherein the method comprises the step of administering to the subject an effective amount of the antibody or the antigen-binding fragment thereof as described in any one embodiment above or the pharmaceutical composition as described in any one embodiment above. In some embodiments, the method as described above further comprises the step of coadministering to the subject one or more therapies comprising surgical treatment and/or radiation therapy and/or administration of one or more other anti-tumor agents, wherein the other anti-tumor agents are selected from chemotherapeutic agents or immune checkpoint inhibitors. The chemotherapeutic agents are selected from alkylating agents, alkyl sulfonates, aziridines, ethylenimines and methylamelamines, acetogenin, δ-9-tetrahydrocannabinol, β-lapachone, lapachol, colchicines, betulinic acid, camptothecin, bryostatin, pemetrexed, podophyllotoxin, podophyllinic acid, teniposide, cryptophycins, dolastatin, eleutherobin, spongistatin, nitrogen mustards, cholophosphamide, estramustine, ifosfamide, mechlorethamine, mechlorethamine oxide hydrochloride, melphalan, novembichin, phenesterine, prednimustine, trofosfamide, uracil mustard, nitrosoureas, antibiotics, detorubicin, 6-diaza-5-oxo-L-norleucine, doxorubicin, epirubicin, esorubicin, idarubicin, marcellomycin, mitomycins, antimetabolites, folic acid analogs, purine analogs, pyrimidine analogs, c-Kit inhibitors, anti-adrenals, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, etoglucid, gallium nitrate, hydroxyurea, lentinan, lonidainine, maytansinoids, mopidanmol, nitraerine, pentostatin, phenamet, pirarubicin, losoxantrone, 2-ethylhydrazide, procarbazine, razoxane, rhizoxin, sizofiran, spirogermanium, tenuazonic acid, triaziquone, 2,2',2"-trichlorotriethylamine, trichothecenes, urethan, vindesine, dacarbazine, mannomustine, mitobronitol, mitolactol, pipobroman, gacytosine, arabinoside, thiotepa, toxoids, chloranbucil, 6-thioguanine, mercaptopurine, methotrexate, platinum analogue, etoposide (VP-16), ifosfamide, mitoxantrone, vincristine, oxaliplatin, leucovorin, vinorelbine, novantrone, edatrexate, daunomycin, aminopterin, ibandronate, difluoromethylomithine (DFMO), retinoids, pharmacologically acceptable salts, acids or derivatives of any of the aforementioned substances, and combinations of two or more of the aforementioned substances, such as CHOP (an abbreviation for combination therapy of cyclophosphamide, doxorubicin, vincristine and prednisolone) and FOLFOX (an abbreviation for therapeutic regimen of oxaliplatin (ELOXATINTM) in combination with 5-FU and leucovorin). Preferably, the chemotherapeutic agents comprise paclitaxel. In some embodiments of the present invention, the present invention provides the method for treating or preventing cancer as described above, wherein the method comprises administering to the subject an effective amount of the anti-DKK1 antibody or the antigen-binding fragment thereof as described in any one embodiment above or the pharmaceutical composition as described in any one embodiment above. In some embodiments, the method as described above further comprises coadministering to the subject one or more therapies comprising administering paclitaxel. In some embodiments of the present invention, the anti-DKK1 antibody or the antigen-binding fragment thereof can be administered simultaneously or asynchronously with paclitaxel in the method for treating or preventing cancer as described above. In some embodiments of the present invention, the anti-DKK1 antibody or the antigen-binding fragment thereof is administered once or twice per week in the method for treating or preventing cancer as described above. In some embodiments of the prevent invention, paclitaxel is administered once or twice per week in the method for treating or preventing cancer as described above.

In another aspect, the present invention provides use of the anti-DKK1 antibody or the antigen-binding fragment thereof described herein in producing or preparing a medicament for treating related diseases or disorders as mentioned above.

In another aspect, the present invention provides use of the anti-DKK1 antibody or the antigen-binding fragment thereof described herein in combination with a tubulin inhibitor in preparing a medicament for treating cancer in a subject. Methods for Diagnosis and Detection

In certain embodiments, any of the anti-DKK1 antibodies or the antigen-binding fragments thereof provided herein can be used to detect the presence of DKK1 in a biological sample. The term "detection" as used herein includes quantitative or qualitative detection. In certain embodiments, the biological sample is blood, serum, or other liquid samples of biological origin. In certain embodiments, the biological sample includes cells or tissues.

The present invention includes any combinations of the specific embodiments described. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entireties for all purposes.

In the present invention, the terms "first" (e.g., first product, first antibody, first functional protein domain, etc.) and "second" (e.g., second product, second antibody, second functional protein domain, etc.) are used for the sake of distinction in reference or clarity in expression and do not have typical sequential meanings, unless otherwise specified.

In the present invention, the term "other anti-tumor agent" refers to an anti-tumor agent except the anti-DKK1 antibody or the antigen-binding fragment thereof of the present invention and the conjugate of the present invention; preferably, the other anti-tumor agent is a chemotherapeutic agent or an immune checkpoint inhibitor, wherein preferably, the chemotherapeutic agent is a tubulin inhibitor and preferably paclitaxel.

The following abbreviations are used in the present invention:
CD CHO medium stands for a chemically defined medium for China hamster ovary cell;
PEI stands for polyethyleneimine;
BSA stands for bovine serum albumin.

### Examples

The amino acid sequence of the heavy and light chains of DKN-01 is set forth in SEQ ID NO: 108 and SEQ ID NO: 109;
The amino acid sequence of the heavy and light chains of BHQ-880 is set forth in SEQ ID NO: 110 and SEQ ID NO: 111.
Example 1: Preparation and Detection of Recombinant Antigen Protein DKK1-hFC
The antigen for animal immunization was a fusion protein of human DKK1 and human IgG1 Fc. GENEWIZ was entrusted to carry out gene synthesis for the designed sequence and the synthesized plasmid was transformed into a top 10 strain. The strain was inoculated into an LB culture medium at a ratio of 1:100 for culturing and collected after 18 h. The supernatant was discarded, and the plasmid was extracted using an endotoxin-controlled plasmid extraction kit (TIANGEN). CHO-K1 cells (engineered at the genomic level to be suitable for transient expression) were expanded by subculturing in a CD CHO medium. The cells were diluted to a density of 1.5-2.0 × 10⁶/mL the day before transfection, and the cells were used for transfection the following day when the cell density reached about 3.5 × 10⁶/mL. One tenth of transfection volume of medium was added first, then 1-2 µg/mL transfection volume of the above-described synthesized plasmid, and finally 3-14 µg/mL transfection volume of PEI. After being mixed well and incubated at room temperature for no more than 30 min, the transfection mixture was slowly added to the cells. The cells were cultured in a shaker under the conditions of 36.5 °C, 120 rpm, and 7% CO₂. The culturing was performed for 6-10 days after the transfection, and the medium supplementation was performed once every two days.

After the culturing was completed, the precipitate was discarded by centrifugation at 1000 g for 10 min, and then the cell supernatant was collected by centrifugation at 12000 g for 30 min. The cell supernatant was sterilely filtered and then purified using an AKTA Avant purifier. A column filled with the mabselect sure LX was first subjected to CIP with 0.1 M NaOH for 15-20 min and then equilibrated with 3-5 column volumes of PBS before sample loading. After the sample loading was completed, the column was rinsed with an affinity chromatography elution buffer. Finally, the target protein was eluted with a pH 3.6 acetic acid-sodium acetate buffer, and the sample was neutralized with 1 M Tris buffer. After the identification of the size and purity of the protein by an SDS-PAGE method, it was determined that the quality of the prepared antigen was acceptable.

### Example 2: Preparation, Screening and Sequencing of Hybridoma Cells

### 2.1 Animal immunization

The antigen DKK1-hFc fusion protein prepared in Example 1 was used to immunize 5 BALB/c female mice by means of bilateral footpad immunization. The dose for the primary immunization was 50 µg/mouse, and the dose for 6 booster immunizations was 25 µg/mouse. The booster immunization was performed every other week.

### 2.2 Cell fusion

After the last booster immunization, the inguinal lymph nodes, the popliteal lymph nodes and the spleens of the mice were collected and ground in normal saline, and then the suspension rich in lymphocytes was collected and fused with mouse myeloma cell Sp2/0 using the conventional electrotransfection method. The fusion products were cultured in the DMEM complete medium containing 1:50 HAT (Hypoxanthine, Aminopterin and Thymidine) for 5 days to screen for successfully fused cells, and then the culturing was carried out in the DMEM complete medium containing 1:50 HT until the screening was completed. The formula of the DMEM complete medium was as follows: 15% FBS + 1:50 L-glutamine + 100 U/mL penicillin-streptomycin + 1:100 OPI (Oxaloacetate, Pyruvate, and Insulin). The incubator parameter conditions were 8% CO₂ and 37 °C.

### 2.3 Screening of hybridoma cells

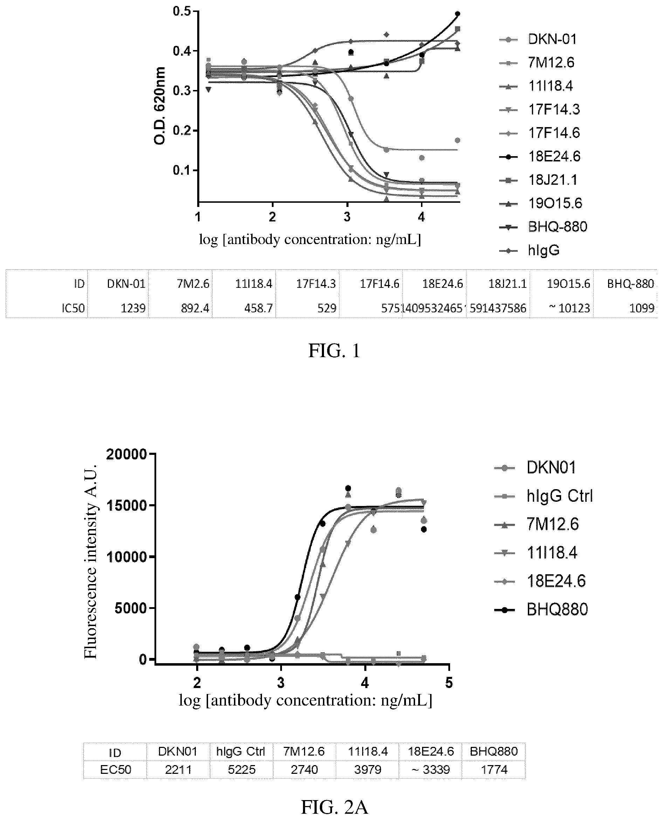

From 11520 strains of different polyclonal hybridoma cells, 15 strains of polyclonal hybridoma cells secreting antibodies capable of competing with LPR6 for binding to DKK1 were obtained by competitive ELISA, with 3 strains showing a weak blocking effect and 12 strains showing a strong blocking effect. The 15 strains of antibodies were subcloned and screened by competitive ELISA to obtain 7 strains of monoclonal antibodies (FIG. 1).

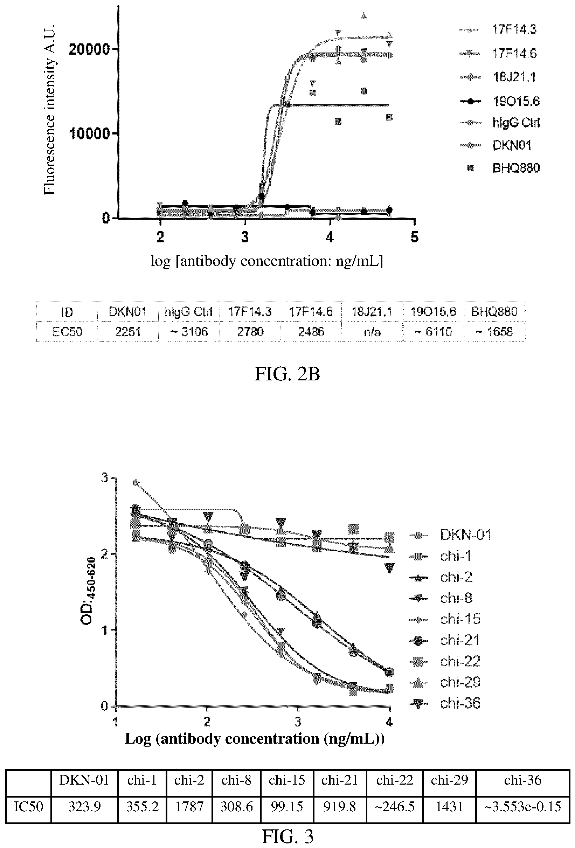

The cellular biological activity of the DKK1 antibodies was tested using target cells 293 TOP overexpressing LRP5/6 and comprising the luciferase reporter system and recombinant Wnt3a (R&D systems, cat # 5036-WN), as well as human DKK1 (Sino Biological, cat # 10170-H08H) protein. The target cells 293 TOP were placed into a 96-well flat-bottomed white plate (corning, cat # 3917) at 50,000 cells per well for incubation overnight. The supernatant was then discarded, and the gradiently diluted antibodies to be tested were each added for pre-incubation at 37 °C for 0.5 h. After the pre-incubation was completed, the diluted Wnt3a (R&D systems, cat # 5036-WN) and human DKK1 protein, at working concentrations of 0.05 µg/mL and 0.1 µg/mL, respectively, were added into the mixture containing the cells and antibody for co-incubation at 37 °C for 6-8 h. Finally, the luciferase substrate One-Lite (Vazyme, cat # DD1203-03-AB) was added and the chemiluminescence values of the hybridoma antibodies were measured. Among them, 7 strains of antibodies showed different degrees of recovery of the Wnt signaling pathway as detected by the luciferase reporter gene method (FIGs. 2A and 2B).

7 strains of hybridoma cells were sent to GenScript Biotech Corporation for sequencing, among which two strains of cells had completely identical sequences, and 6 strains of hybridoma antibodies were finally obtained and named 7M2.6, 11118.4, 17F14.3, 18E24.6, 18J21.1 and 19O15.6, respectively.

### 2.4 Acquisition of variable region sequences of candidate antibodies (Kabat definition scheme)

The DNA sequences of the variable regions of the mouse antibodies expressed by the candidate hybridoma cells were determined using a degenerate primer-based PCR method. Briefly, hybridoma cell strains were separately expanded, cells were harvested by centrifugation at 1000 rpm, and total RNA was extracted with Trizol. Using the total RNA as a template, a first-strand cDNA was synthesized. DNA sequences of corresponding variable regions were amplified by PCR using the first-strand cDNA as a subsequent template. The PCR primers used were based on an Ig-primer set. After isolating and purifying PCR products and sequencing the amplified products, the coding sequences of the heavy chain variable regions and light chain variable regions of the candidate hybridoma antibodies were obtained, and thus the amino acid sequences of the heavy chain variable regions and light chain variable regions of the hybridoma antibodies were obtained, which are as shown in Table 1 below.

**Table 1. Amino acid sequences of variable regions of hybridoma antibodies\**

| Hybridoma antibody | Variable region | Amino acid sequence |
|---|---|---|
| 7M2.6 | 1VH | SEQ ID NO: 37 |
| | 1VL | SEQ ID NO: 38 |
| 11I18.4 | 2VH | SEQ ID NO: 39 |
| | 2VL | SEQ ID NO: 40 |
| 17F14.3 | 3VH | SEQ ID NO: 41 |
| | 3VL | SEQ ID NO: 42 |
| 18E24.6 | 5VH | SEQ ID NO: 43 |
| | 5VL | SEQ ID NO: 44 |
| 18J21.1 | 6VH | SEQ ID NO: 45 |
| | 6VL | SEQ ID NO: 46 |
| 19O15.6 | 7VH | SEQ ID NO: 47 |
| | 7VL | SEQ ID NO: 48 |

The NCBI Ig-Blast (http://www.ncbi.nlm.nih.gov/projects/igblast/) was used to search for consensus sequences in germline and rearranged Ig variable region sequence databases. Complementarity determining regions (CDRs) were identified by sequence annotation and by Internet-based sequence analysis (http://www.Imgt.org/IMGT_vquest/share/textes/index.html and http://www.ncbi.nlm.nih.gov/igblast/) based on Kabat (Wu, T.T and Kabat, E.A. 1970 J. Exp. Med., 132: 211-250) and IMGT systems (Lefranc M.-P. et al., 1999 Nucleic Acids Research, 27, 209-212). The CDRs are as shown in Table 2 below.

**Table 2. Amino acid sequences of CDR regions of hybridoma antibodies (Kabat definition scheme)**

| Hybridoma antibody | CDR | Amino acid sequence |
|---|---|---|
| 7M2.6 | HCDR1 | SEQ ID NO: 1 |
| | HCDR2 | SEQ ID NO: 2 |
| | HCDR3 | SEQ ID NO: 78 |
| | LCDR1 | SEQ ID NO: 4 |
| | LCDR2 | SEQ ID NO: 5 |
| | LCDR3 | SEQ ID NO: 6 |
| 11I18.4 | HCDR1 | SEQ ID NO: 7 |
| | HCDR2 | SEQ ID NO: 8 |
| | HCDR3 | SEQ ID NO: 9 |
| | LCDR1 | SEQ ID NO: 10 |
| | LCDR2 | SEQ ID NO: 11 |
| | LCDR3 | SEQ ID NO: 12 |
| 17F14.3 | HCDR1 | SEQ ID NO: 13 |
| | HCDR2 | SEQ ID NO: 14 |
| | HCDR3 | SEQ ID NO: 15 |
| | LCDR1 | SEQ ID NO: 16 |
| | LCDR2 | SEQ ID NO: 17 |
| | LCDR3 | SEQ ID NO: 18 |
| 18E24.6 | HCDR1 | SEQ ID NO: 19 |
| | HCDR2 | SEQ ID NO: 20 |
| | HCDR3 | SEQ ID NO: 21 |
| | LCDR1 | SEQ ID NO: 22 |
| | LCDR2 | SEQ ID NO: 23 |
| | LCDR3 | SEQ ID NO: 24 |
| 18J21.1 | HCDR1 | SEQ ID NO: 25 |
| | HCDR2 | SEQ ID NO: 26 |
| | HCDR3 | SEQ ID NO: 27 |
| | LCDR1 | SEQ ID NO: 28 |
| | LCDR2 | SEQ ID NO: 29 |
| | LCDR3 | SEQ ID NO: 30 |
| 19O15.6 | HCDR1 | SEQ ID NO: 31 |
| | HCDR2 | SEQ ID NO: 32 |
| | HCDR3 | SEQ ID NO: 33 |
| | LCDR1 | SEQ ID NO: 34 |
| | LCDR2 | SEQ ID NO: 35 |
| | LCDR3 | SEQ ID NO: 36 |

### Example 3: Design, Construction and Expression of Chimeric Antibodies

According to the sequencing results in Example 2, the light chain variable regions and heavy chain variable regions of the anti-huDKK1 murine antibodies 7M2.6, 11I18.4, 17F14.3, 18E24.6, 18J21.1 and 19O15.6 (sequences shown in Table 3 below) were selected. The heavy chain variable regions were each constructed into the hIgG4 heavy chain constant region (the amino acid sequence of the heavy chain constant region is set forth in SEQ ID NO: 112), and the light chain variable regions were each constructed into hIg κ chain (the amino acid sequence of the light chain constant region is set forth in SEQ ID NO: 113). The chimeric heavy and chain variable regions were randomly combined to form 36 strains of chimeric antibodies, as shown in Table 3 below.

**Table 3. Numbering of chimeric antibodies (chi1-chi36) and combinations of their heavy variable regions and light chain variable regions**

| | 1VL | 2VL | 3VL | 5VL | 6VL | 7VL |
|---|---|---|---|---|---|---|
| 1VH | chi-1 | chi-7 | chi-13 | chi-19 | chi-25 | chi-31 |
| 2VH | chi-2 | chi-8 | chi-14 | chi-20 | chi-26 | chi-32 |
| 3VH | chi-3 | chi-9 | chi-15 | chi-21 | chi-27 | chi-33 |
| 5VH | chi-4 | chi-10 | chi-16 | chi-22 | chi-28 | chi-34 |
| 6VH | chi-5 | chi-11 | chi-17 | chi-23 | chi-29 | chi-35 |
| 7VH | chi-6 | chi-12 | chi-18 | chi-24 | chi-30 | chi-36 |

CHO-K1 cells (engineered at the genomic level to be suitable for transient expression) were expanded by subculturing in a CD CHO medium. The cells were diluted to a density of 1.5-2.0 × 10⁶/mL the day before transfection, and the cells were used for transfection the following day when the cell density reached about 3.5 × 10⁶/mL. One tenth of transfection volume of medium was added first, then 1-2 µg/mL transfection volume of the plasmid encoding the chimeric antibody, and finally 3-14 µg/mL transfection volume of PEI. After being mixed well and incubated at room temperature for no more than 30 min, the transfection mixture was slowly added to the cells. The cells were cultured in a shaker under the conditions of 36.5 °C, 120 rpm, and 7% CO₂. The culturing was performed for 6-10 days after the transfection, and the medium supplementation was performed once every two days.

After the culturing was completed, the precipitate was discarded by centrifugation at 1000 g for 10 min, and then the cell supernatant was collected by centrifugation at 12000 g for 30 min. The cell supernatant was sterilely filtered and then purified using an AKTA Avant purifier. A column filled with the mabselect sure LX was first subjected to CIP with 0.1 M NaOH for 15-20 min and then equilibrated with 3-5 column volumes of PBS before sample loading. After the sample loading was completed, the column was rinsed with an affinity chromatography elution buffer. Finally, the target protein was eluted with a pH 3.6 acetic acid-sodium acetate buffer, and the sample was neutralized with 1 M Tris buffer. After the identification of the size and purity of the proteins by an SDS-PAGE method, it was determined that the quality of the prepared chimeric antibodies was acceptable.

### Example 4. Screening of Chimeric Antibodies

### 4.1 Screening at protein level

Detection of the binding activity of the chimeric antibodies for human DKK1 by ELISA: After the plate was coated with 2.0 µg/mL human DKK1 antigen (Sino Biological, 10170-H08H) for 90 min and then blocked with 2% BSA for 90 min, the gradiently diluted anti-DKK1 chimeric antibodies and positive control antibody DKN-01 (starting at 1 µg/mL, diluted in a 2.5-fold gradient to 1.64 ng/mL, with a total of 8 concentrations) were added, and the mixture was incubated for 60 min. After the plate was washed, the 5000-fold diluted HRP-conjugated goat anti-human IgG (Fc specific) (Sigma, A0170) secondary antibody was added for incubation, and 0.1 mg/mL TMB (Sigma, T2885) was added for color development. Finally, the reaction was stopped with a 2 M hydrochloric acid solution. The plate was read at 450 nm/620 nm using a Thermo Scientific microplate reader, and the EC₅₀ values of the chimeric antibodies (FIG. 3) were obtained by fitting using a four-parameter logistic regression (4PL) model. The relative binding activities of the chimeric antibodies were compared, and the VHs and VLs of the chi-1, chi-8 and chi-15 molecules with superior activity were selected for the subsequent construction of humanized antibodies.

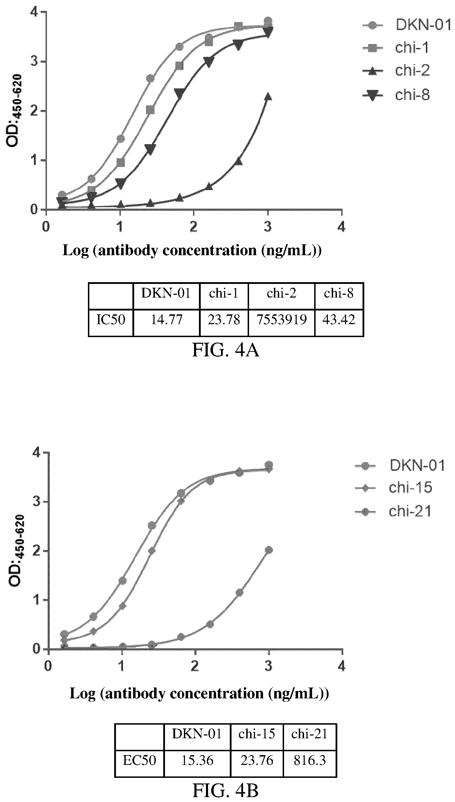

Detection of the inhibition of the chimeric antibodies on the binding of LRP6 (629-1244) to DKK1 by ELISA: The plate was coated with 5.0 µg/mL human DKK1 antigen (Sino Biological, 10170-H08H) for 90 min and blocked with 2% BSA for 90 min. The anti-DKK1 chimeric antibodies and positive control antibody DKN-01 were diluted with human LRP6 mFc (629-1244) (Suzhou Junmeng) (starting at 10 µg/mL, diluted in a 2.5-fold gradient to 16.38 ng/mL, with a total of 8 concentrations), and the mixture was incubated for 60 min. After the plate was washed, the 5000-fold diluted HRP-conjugated goat anti-mouse IgG (Fc specific) (Sigma, A2554) was added for incubation for 30 min. After the plate was further washed, 0.1 mg/mL TMB (Sigma, T2885) was added at 100 µL per well for color development for 10 min. Finally, a 2 M hydrochloric acid solution was added to each well to stop the reaction. The plate was read at 450 nm/620 nm. The IC₅₀ values of the chimeric antibodies (FIGs. 4A-4B) were obtained by fitting using a four-parameter logistic regression (4PL) model, and the relative competitive inhibitory activities of the chimeric antibodies were compared.

### 4.2 Screening at cellular level

The chi1 supernatant and the chi15 supernatant were fermentation broth supernatants and were not purified, and chi1, chi8, chi15 and chi21 were purified protein samples. Reference is made to the previous Example 3 for the preparation of fermentation broth supernatants and protein samples.

The cellular biological activity of the hDKK1 chimeric antibodies was tested using target cells 293 TOP overexpressing LRP5/6 and comprising the luciferase reporter system and recombinant Wnt3a (R&D systems, cat # 5036-WN), as well as human DKK1 (Sino Biological, cat # 10170-H08H) protein.

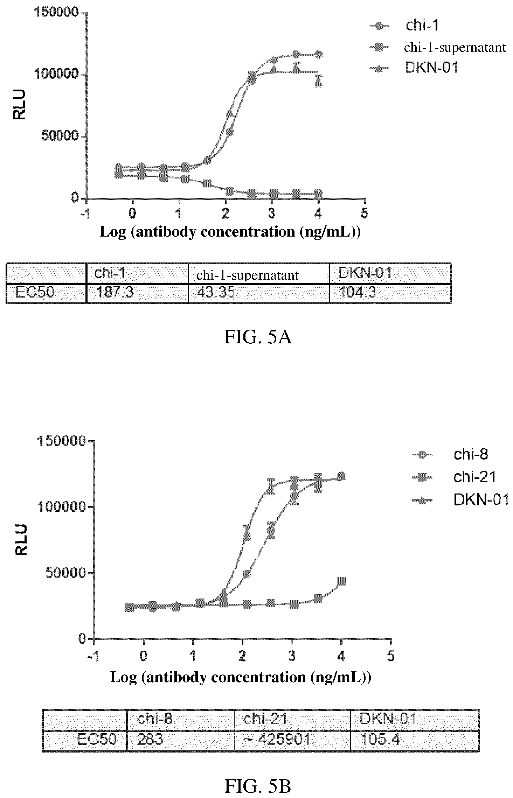

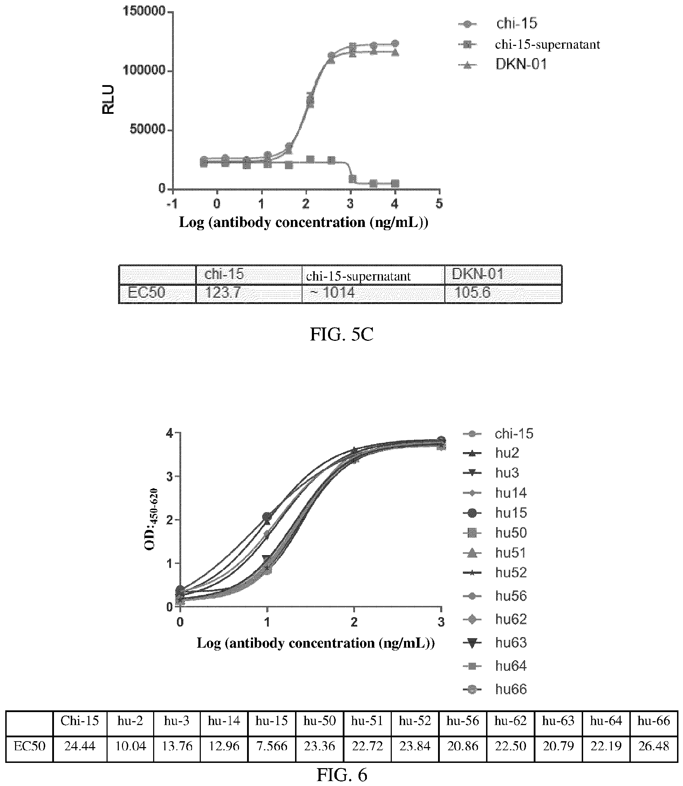

The target cells 293 TOP were placed into a 96-well flat-bottomed white plate (corning, cat # 3917) at 50,000 cells per well for incubation overnight. The supernatant was then discarded, and the gradiently diluted antibodies to be tested were each added for pre-incubation at 37 °C for 0.5 h. After the pre-incubation was completed, the diluted Wnt3a (R&D systems, cat # 5036-WN) and human DKK1 protein, at working concentrations of 0.05 µg/mL and 0.1 µg/mL, respectively, were added into the mixture containing the cells and antibody for co-incubation at 37 °C for 6-8 h. Finally, the luciferase substrate One-Lite (Vazyme, cat # DD1203-03-AB) was added and the chemiluminescence values of the chimeric antibodies were measured (FIGs. 5A-5C).

The screening of the chimeric antibodies of Example 4 yielded 3 antibodies chi1, chi8 and chi15 with the best *in vitro* activity for subsequent humanization.

### Example 5: Humanization of Chimeric Antibodies

Human IgG genes homologous to the DNA sequences of the murine antibody variable regions were retrieved in the human immunoglobulin gene database of the NCBI (http://www.ncbi.nlm.nih.gov/igblast/). The amino acid sequences of CDRs in the variable regions were then determined by the Kabat numbering system or the IMGT numbering system. In principle, human IGHV (immunoglobulin heavy chain constant region) and IGKV (immunoglobulin light chain constant region) with high homology to the variable regions of the murine antibody were selected as templates for humanization, and antibody variable regions were humanized by CDR grafting.

The humanization of the chimeric antibodies comprised the following steps: 1. comparing the gene sequence of the antibody secreted by each hybridoma cell with the gene sequence of the human embryonic antibody to find out a sequence with high homology; 2. analyzing and inspecting HLA-DR affinity and selecting a human embryonic framework region sequence with low affinity; 3. analyzing the framework region amino acid sequences of the variable regions and their periphery by using a computer simulation technology and applying molecular docking to investigate their spatial and stereo combination modes; analyzing the key amino acids that interacted with huDKK1 and maintained the spatial framework in the gene sequence of the antibody secreted by each hybridoma cell by calculating electrostatic force, Van der Waals' force, hydrophilicity and hydrophobicity, and entropy value and grafting them to the selected human embryonic gene framework regions; mapping the amino acid sites of the framework regions which must be retained; and 4. performing back mutation on residues that were buried, residues that directly interacted with CDR regions, and residues that had a significant effect on the conformation of VL and VH, on the basis of the three-dimensional structure of the murine antibody, and optimizing amino acid residues that led to chemical instability of the CDR regions of the antibodies.

Finally, the humanization of the No. 1 chimeric antibody molecule (chi-1) resulted in 5 heavy chains and 4 light chains (after humanization, HCDR3 was mutated from SEQ ID NO: 78 to SEQ ID NO: 3), the humanization of the No. 2 chimeric antibody molecule (chi-8) resulted in 5 heavy chains and 5 light chains, and the humanization of the No. 3 chimeric antibody molecule (chi-15) resulted in 6 heavy chains and 4 light chains. The heavy and light chains of the 3 molecules were randomly combined to obtain 69 strains of humanized molecules (CDR sequences were defined according to the Kabat definition scheme), and their combinations are shown in the following Tables 4a-4c and 5a-5c.

**Table 4a. Numbering of chi-1-based humanized antibodies and combinations of their heavy chain variable regions and light chain variable regions**

| | 1VH-1 | 1VH-2 | 1VH-3 | 1VH-4 | 1VH-5 |
|---|---|---|---|---|---|
| | SEQ ID NO: 49 | SEQ ID NO: 50 | SEQ ID NO: 51 | SEQ ID NO: 52 | SEQ ID NO: 53 |
| 1VL-1 | hu1 | hu5 | hu9 | hu13 | hu17 |
| SEQ ID NO: 54 | | | | | |
| 1VL-2 | hu2 | hu6 | hu10 | hu14 | hu18 |
| SEQ ID NO: 55 | | | | | |
| 1VL-3 | hu3 | hu7 | hu11 | hu15 | hu19 |
| SEQ ID NO: 56 | | | | | |
| 1VL-4 | hu4 | hu8 | hu12 | hu16 | hu20 |
| SEQ ID NO: 57 | | | | | |

**Table 4b. Numbering of chi-8-based humanized antibodies and combinations of their heavy chain variable regions and light chain variable regions**

| | 2VH-1 | 2VH-2 | 2VH-3 | 2VH-4 | 2VH-5 |
|---|---|---|---|---|---|
| | SEQ ID NO: 58 | SEQ ID NO: 59 | SEQ ID NO: 60 | SEQ ID NO: 61 | SEQ ID NO: 62 |
| 2VL-1 | hu21 | hu26 | hu31 | hu36 | hu41 |
| SEQ ID NO: 63 | | | | | |
| 2VL-2 | hu22 | hu27 | hu32 | hu37 | hu42 |
| SEQ ID NO: 64 | | | | | |
| 2VL-3 | hu23 | hu28 | hu33 | hu38 | hu43 |
| SEQ ID NO: 65 | | | | | |
| 2VL-4 | hu24 | hu29 | hu34 | hu39 | hu44 |
| SEQ ID NO: 66 | | | | | |
| 2VL-5 | hu25 | hu30 | hu35 | hu40 | hu45 |
| SEQ ID NO: 67 | | | | | |

**Table 4c. Numbering of chi-15-based humanized antibodies and combinations of their heavy chain variable regions and light chain variable regions**

| | 3VH-1 | 3VH-2 | 3VH-3 | 3VH-4 | 3VH-5 | 3VH-6 |
|---|---|---|---|---|---|---|
| | SEQ ID NO: 68 | SEQ ID NO: 69 | SEQ ID NO: 70 | SEQ ID NO: 71 | SEQ ID NO: 72 | SEQ ID NO: 73 |
| 3VL-1 | hu46 | hu50 | hu54 | hu58 | hu62 | hu66 |
| SEQ ID NO: 74 | | | | | | |
| 3VL-2 | hu47 | hu51 | hu55 | hu59 | hu63 | hu67 |
| SEQ ID NO: 75 | | | | | | |
| 3VL-3 | hu48 | hu52 | hu56 | hu60 | hu64 | hu68 |
| SEQ ID NO: 76 | | | | | | |
| 3VL-4 | hu49 | hu53 | hu57 | hu61 | hu65 | hu69 |
| SEQ ID NO: 77 | | | | | | |

**Table 5a. Numbering of chi-1-based humanized antibodies and combinations of their heavy chains and light chains**

| | 1HC-1 | 1HC-2 | 1HC-3 | 1HC-4 | 1HC-5 |
|---|---|---|---|---|---|
| | SEQ ID NO: 79 | SEQ ID NO: 80 | SEQ ID NO: 81 | SEQ ID NO: 82 | SEQ ID NO: 83 |
| 1LC-1 | hu1 | hu5 | hu9 | hu13 | hu17 |
| SEQ ID NO: 84 | | | | | |
| 1LC-2 | hu2 | hu6 | hu10 | hu14 | hu18 |
| SEQ ID NO: 85 | | | | | |
| 1LC-3 | hu3 | hu7 | hu11 | hu15 | hu19 |
| SEQ ID NO: 86 | | | | | |
| 1LC-4 | hu4 | hu8 | hu12 | hu16 | hu20 |
| SEQ ID NO: 87 | | | | | |

**Table 5b. Numbering of chi-8-based humanized antibodies and combinations of their heavy chains and light chains**

| | 2HC-1 | 2HC-2 | 2HC-3 | 2HC-4 | 2HC-5 |
|---|---|---|---|---|---|
| | SEQ ID NO: 88 | SEQ ID NO: 89 | SEQ ID NO: 90 | SEQ ID NO: 91 | SEQ ID NO: 92 |
| 2LC-1 | hu21 | hu26 | hu31 | hu36 | hu41 |
| SEQ ID NO: 93 | | | | | |
| 2LC-2 | hu22 | hu27 | hu32 | hu37 | hu42 |
| SEQ ID NO: 94 | | | | | |
| 2LC-3 | hu23 | hu28 | hu33 | hu38 | hu43 |
| SEQ ID NO: 95 | | | | | |
| 2LC-4 | hu24 | hu29 | hu34 | hu39 | hu44 |
| SEQ ID NO: 96 | | | | | |
| 2LC-5 | hu25 | hu30 | hu35 | hu40 | hu45 |
| SEQ ID NO: 97 | | | | | |

**Table 5c. Numbering of chi-15-based humanized antibodies and combinations of their heavy chains and light chains**

| | 3HC-1 | 3HC-2 | 3HC-3 | 3HC-4 | 3HC-5 | 3HC-6 |
|---|---|---|---|---|---|---|
| | SEQ ID NO: 98 | SEQ ID NO: 99 | SEQ ID NO: 100 | SEQ ID NO: 101 | SEQ ID NO: 102 | SEQ ID NO: 103 |
| 3LC-1 | hu46 | hu50 | hu54 | hu58 | hu62 | hu66 |
| SEQ ID NO: 104 | | | | | | |
| 3LC-2 | hu47 | hu51 | hu55 | hu59 | hu63 | hu67 |
| SEQ ID NO: 105 | | | | | | |
| 3LC-3 | hu48 | hu52 | hu56 | hu60 | hu64 | hu68 |
| SEQ ID NO: 106 | | | | | | |
| 3LC-4 | hu49 | hu53 | hu57 | hu61 | hu65 | hu69 |
| SEQ ID NO: 107 | | | | | | |

### Example 6: Preparation of Humanized Antibodies

GenScript Biotech Corporation was entrusted to carry out gene synthesis for the plasmids encoding the heavy chains and light chains of the 69 humanized antibodies hu1-hu69. After the synthesized plasmids and strains were received, the strains were transferred to an LB medium for overnight culturing, and plasmid extraction was performed with the Maxi Plasmid Kit (TIANGEN).

CHO-K1 cells (engineered at the genomic level to be suitable for transient expression) were expanded by subculturing in a CD CHO medium. The cells were diluted to a density of 1.5-2.0 × 10⁶/mL the day before transfection, and the cells were used for transfection the following day when the cell density reached about 3.5 × 10⁶/mL. One tenth of transfection volume of medium was added first, then 1-2 µg/mL transfection volume of the plasmid encoding the chimeric antibody, and finally 3-14 µg/mL transfection volume of PEI. After being mixed well and incubated at room temperature for no more than 30 min, the transfection mixture was slowly added to the cells, and the final volume was about 40 mL. The cells were cultured in a shaker under the conditions of 36.5 °C, 120 rpm, and 7% CO₂. The culturing was performed for 6-10 days after the transfection, and the medium supplementation was performed once every two days.

After the culturing was completed, the precipitate was discarded by centrifugation at 1000 g for 10 min, and then the cell supernatant was collected by centrifugation at 12000 g for 30 min and sterilely filtered. The 69 strains of humanized antibodies were purified by the same method as that for the hybridoma antibodies in Example 2. After purification, the size and purity of the antibody proteins were identified by SDS-PAGE, and the purity of the proteins was detected by HPLC-SEC. The antibodies were used for later experiments.

### Example 7: Screening of Humanized Antibodies

### 7.1 Screening at protein level

Detection of the binding of the humanized antibodies to DKK1 by ELISA: The plate was coated with 2.0 µg/mL human DKK1 antigen (Sino Biological, 10170-H08H) for 90 min and then blocked with 2% BSA for 90 min. After the plate was washed, the gradiently diluted anti-DKK1 humanized antibodies (starting at 1 µg/mL, diluted in a 10-fold gradient to 1 ng/mL, with a total of 4 concentrations) were each added, and the mixture was incubated for 60 min. After the plate was further washed, the 5000-fold diluted HRP-conjugated goat anti-human IgG (Fc specific) (Sigma, A0170) secondary antibody was added for incubation for 30 min, and 0.1 mg/mL TMB (Sigma, T2885) was added at 100 µL per well for color development for 10 min. Finally, a 2 M hydrochloric acid solution was added at 50 µL per well to stop the reaction. The plate was read at 450 nm/620 nm using a Thermo Scientific microplate reader. The EC₅₀ values of the humanized antibodies were obtained by fitting using a four-parameter logistic regression (4PL) model. Some of the obtained humanized antibodies exhibited improved ability to bind to human DKK1 compared with the chimeric antibody chi-15 (FIG. 6).

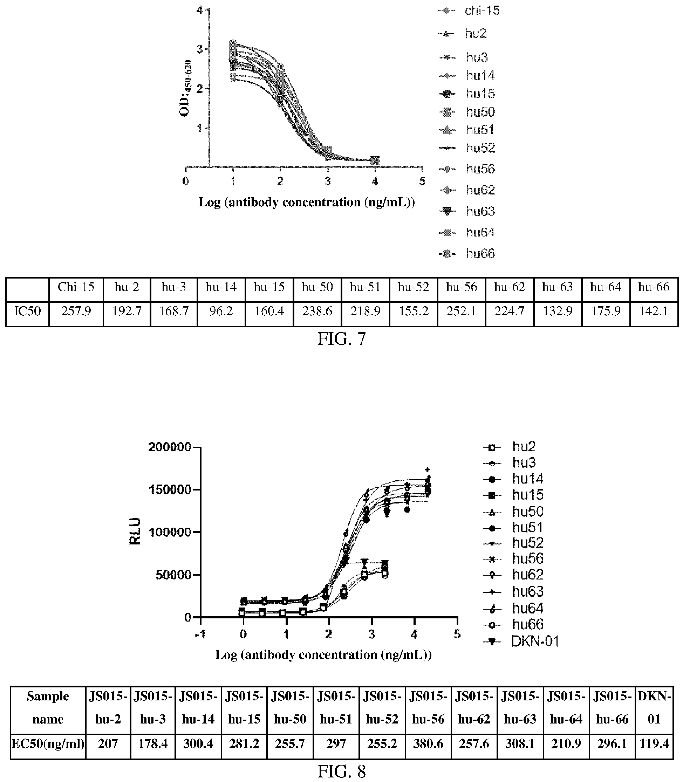

Detection of the inhibition of the humanized antibodies on the binding of LRP6 (629-1244) to DKK1 by ELISA: The plate was coated with 5.0 µg/mL human DKK1 antigen (Sino Biological, 10170-H08H) for 90 min and then blocked with 2% BSA for 90 min. After the plate was washed, the DKK1 antibodies, i.e., the humanized antibodies and the anti-DKK1 chimeric antibody, were diluted with 10 µg/mL human LRP6 mFc (629-1244) (Suzhou Junmeng) (starting at 10 µg/mL, diluted in a 10-fold gradient to 10 ng/mL, with a total of 4 concentrations), and the mixture was incubated for 60 min. The plate was washed, and a 5000-fold diluted HRP-conjugated goat anti-mouse IgG (Fc specific) (Sigma, A2554) was added at 100 µL per well for incubation for 30 min. After the plate was further washed, a 0.1 mg/mL TMB (Sigma, T2885) was added at 100 µL per well for color development for 10 min. Finally, a 2 M hydrochloric acid solution was added at 50 µL per well to stop the reaction, and the plate was read at 450 nm/620 nm using a Thermo Scientific microplate reader. The IC₅₀ values of the humanized antibodies were obtained by fitting using a four-parameter logistic regression (4PL) model. Some of the obtained humanized antibodies exhibited improved ability to inhibit the binding of DKK1 to LRP6 compared with the chimeric antibody chi-15 (FIG. 7).

### 7.2 Screening at cellular level

The cellular biological activity of the DKK1 antibodies was tested using target cells 293 TOP overexpressing LRP5/6 and comprising the luciferase reporter system and recombinant Wnt3a (R&D systems, cat # 5036-WN), as well as human DKK1 (Sino Biological, cat # 10170-H08H) protein. The target cells 293 TOP were placed into a 96-well flat-bottomed white plate (corning, cat # 3917) at 50,000 cells per well for incubation overnight. The supernatant was then discarded, and the gradiently diluted antibodies to be tested were each added for pre-incubation at 37 °C for 0.5 h. After the pre-incubation was completed, the diluted Wnt3a (R&D systems, cat # 5036-WN) and human DKK1 protein, at working concentrations of 0.05 µg/mL and 0.1 µg/mL, respectively, were added into the mixture containing the cells and antibody for co-incubation at 37 °C for 6-8 h. Finally, the luciferase substrate One-Lite (Vazyme, cat # DD1203-03-AB) was added and the chemiluminescence values of the humanized antibodies were measured. Some of the obtained humanized antibodies improved the recovery degree of the expression of the Wnt3a pathway compared with the chimeric antibody chi-15 (FIG. 8).

The results of the screening at cellular level showed that the molecules with superior activity were hu3, hu2, hu64, hu52 and hu50.

Through the screening of humanized antibodies at protein and cellular levels (FIG. 7, FIG. 8), the molecules with better effect in the two rounds of screening were used for the research of drug effect *in vivo.*

### Example 8: Inhibition of Tumor Growth in Mice by Humanized Anti-DKK1 Antibody

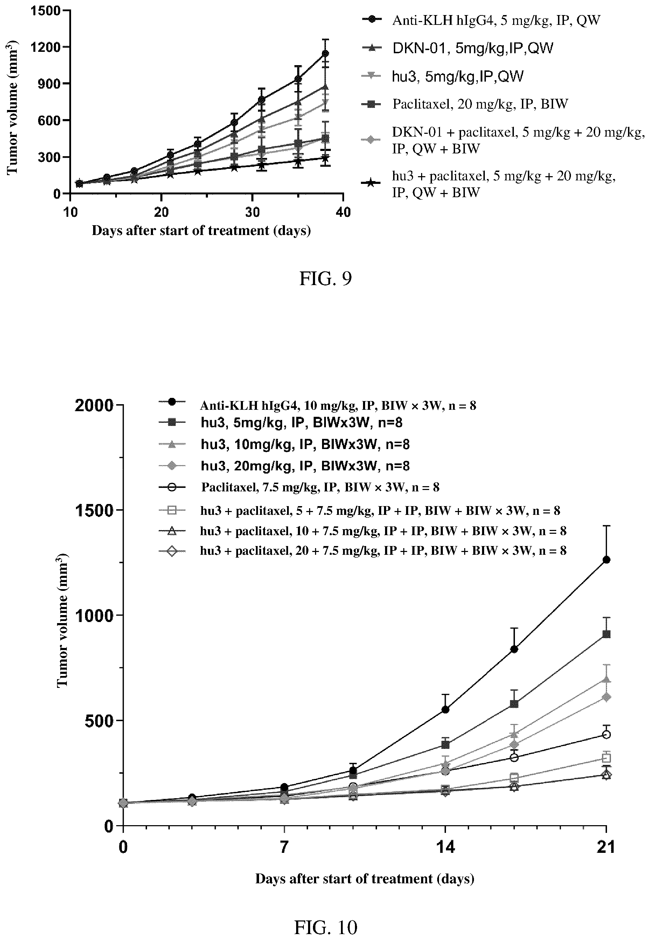

The experimental animals were CB17/SCID female mice, and A549 cells (5 × 10⁶) were subcutaneously inoculated into the mice to establish a subcutaneous transplantation tumor model. The experiment involved the following groups: anti-KLH IgG4 (5 mg/kg) group, DKN-01 (5 mg/kg) group, hu3 (5 mg/kg) group, paclitaxel (20 mg/kg) group, DKN-01 (5 mg/kg) + paclitaxel (20 mg/kg) group, and DKN-01 (5 mg/kg) + hu3 (5 mg/kg) group, where DKN-01 was the positive control antibody and anti-KLH IgG4 was the negative control antibody. The groups each had 6 animals, and the animals in all the groups were subjected to intraperitoneal injection. The negative control, the positive control, and the anti-DKK1 antibody hu3 were all administered once per week for 4 weeks, and paclitaxel was administered twice per week for 4 weeks. The efficacy was evaluated based on tumor growth inhibition (TGI) (FIG. 9).

The experimental results showed that on day 35 after tumor inoculation, the ranking of the groups in a descending order of TGI effect was as follows: the DKN-01 (5 mg/kg) + hu3 (5 mg/kg) group, the paclitaxel (20 mg/kg) group, the DKN-01 (5 mg/kg) + paclitaxel (20 mg/kg) group, the hu3 group, the DKN-01 group and the anti-KLH IgG4 group, with TGI values being 74.48%, 60.48%, 60.04%, 35.29%, 23.27%, and 0%, respectively. The results showed that hu3 exhibited significant tumor inhibition (p = 0.013) at a dose of 5 mg/kg, with an effect superior to that of the positive control DKN-01. In addition, the tumor inhibition effect of the combination of the anti-DKK1 antibody hu3 at the aforementioned concentration with paclitaxel was significantly better than that of hu3 and paclitaxel when used alone and was markedly superior to the combination of the positive control at the same concentration with paclitaxel.

### Example 9: Inhibition of Tumor Growth in Mice by Humanized Anti-DKK1 Antibody

The experimental animals were CB17/SCID male mice, and PC-3 cells (10 × 10⁶) were subcutaneously inoculated in the right back of the mice to establish a subcutaneous transplantation tumor model. The experiment involved the following groups: anti-KLH IgG4 (10 mg/kg) group, hu3 (5 mg/kg) group, hu3 (10 mg/kg) group, hu3 (20 mg/kg) group, paclitaxel (7.5 mg/kg) group, hu3 (5 mg/kg) + paclitaxel (7.5 mg/kg) group, hu3 (10 mg/kg) + paclitaxel (7.5 mg/kg) group and hu3 (20 mg/kg) + paclitaxel (7.5 mg/kg) group, where anti-KLH IgG4 was the negative control antibody. The groups each had 8 animals, and the animals in all the groups were subjected to intraperitoneal injection. The negative control anti-KLH IgG4, paclitaxel and the anti-DKK1 antibody hu3 were all administered twice per week for 3 weeks. The efficacy was evaluated based on tumor growth inhibition (TGI) (FIG. 10).

The experimental results showed that on day 21 after tumor inoculation, the ranking of the groups in a descending order of TGI effect was as follows: the hu3 (20 mg/kg) + paclitaxel (7.5 mg/kg) group, the hu3 (10 mg/kg) + paclitaxel (7.5 mg/kg) group, the hu3 (5 mg/kg) + paclitaxel (7.5 mg/kg) group, the paclitaxel (7.5 mg/kg) group, the hu3 (20 mg/kg) group, the hu3 (10 mg/kg) group, the hu3 (5 mg/kg) group and the anti-KLH IgG4 (10 mg/kg) group, with TGI values being 88.4%, 88.3%, 81.5%, 71.8%, 56.4%, 48.8%, 30.7% and 0%, respectively. The results showed that hu3 exhibited significant tumor inhibition at doses of 5 mg/kg, 10 mg/kg and 20 mg/kg, and the higher doses, the higher TGI values. In addition, the tumor inhibition effect of the combination of hu3 at each of the aforementioned three doses with 7.5 mg/kg paclitaxel was significantly better than that of the hu3 at the three doses and 7.5 mg/kg paclitaxel when used alone, indicating that the co-administration of the anti-DKK1 antibody hu3 and paclitaxel provides a synergistic anti-tumor effect.

Although specific embodiments of the present invention have been described in detail, those skilled in the art will appreciate that various modifications and substitutions can be made to those details according to all the teachings that have been disclosed and these changes shall all fall within the protection scope of the present invention. The full scope of the present invention is given by the appended claims and any equivalents thereof.

## Claims

1. An anti-DKK1 antibody or an antigen-binding fragment thereof,
wherein the anti-DKK1 antibody comprises a heavy chain variable region comprising HCDR1 to HCDR3 and a light chain variable region comprising LCDR1 to LCDR3,
wherein,
the amino acid sequence of HCDR1 is selected from SEQ ID NO: 1, SEQ ID NO: 7, SEQ ID NO: 13, SEQ ID NO: 19, SEQ ID NO: 25, and SEQ ID NO: 31;
the amino acid sequence of HCDR2 is selected from SEQ ID NO: 2, SEQ ID NO: 8, SEQ ID NO: 14, SEQ ID NO: 20, SEQ ID NO: 26, and SEQ ID NO: 32;
the amino acid sequence of HCDR3 is selected from SEQ ID NO: 3, SEQ ID NO: 9, SEQ ID NO: 15, SEQ ID NO: 21, SEQ ID NO: 27, SEQ ID NO: 33, and SEQ ID NO: 78;
the amino acid sequence of LCDR1 is selected from SEQ ID NO: 4, SEQ ID NO: 10, SEQ ID NO: 16, SEQ ID NO: 22, SEQ ID NO: 28, and SEQ ID NO: 34;
the amino acid sequence of LCDR2 is selected from SEQ ID NO: 5, SEQ ID NO: 11, SEQ ID NO: 17, SEQ ID NO: 23, SEQ ID NO: 29, and SEQ ID NO: 35;
the amino acid sequence of LCDR3 is selected from SEQ ID NO: 6, SEQ ID NO: 12, SEQ ID NO: 18, SEQ ID NO: 24, SEQ ID NO: 30, and SEQ ID NO: 36.

2. The anti-DKK1 antibody or the antigen-binding fragment thereof according to claim 1, wherein the anti-DKK1 antibody comprises a heavy chain variable region comprising HCDR1 to HCDR3 and a light chain variable region comprising LCDR1 to LCDR3,
wherein,
for the heavy chain variable region of the antibody,
the amino acid sequence of HCDR1 is set forth in SEQ ID NO: 1, the amino acid sequence of HCDR2 is set forth in SEQ ID NO: 2, and the amino acid sequence of HCDR3 is set forth in SEQ ID NO: 3,
the amino acid sequence of HCDR1 is set forth in SEQ ID NO: 1, the amino acid sequence of HCDR2 is set forth in SEQ ID NO: 2, and the amino acid sequence of HCDR3 is set forth in SEQ ID NO: 78,
the amino acid sequence of HCDR1 is set forth in SEQ ID NO: 7, the amino acid sequence of HCDR2 is set forth in SEQ ID NO: 8, and the amino acid sequence of HCDR3 is set forth in SEQ ID NO: 9,
the amino acid sequence of HCDR1 is set forth in SEQ ID NO: 13, the amino acid sequence of HCDR2 is set forth in SEQ ID NO: 14, and the amino acid sequence of HCDR3 is set forth in SEQ ID NO: 15,
the amino acid sequence of HCDR1 is set forth in SEQ ID NO: 19, the amino acid sequence of HCDR2 is set forth in SEQ ID NO: 20, and the amino acid sequence of HCDR3 is set forth in SEQ ID NO: 21,
the amino acid sequence of HCDR1 is set forth in SEQ ID NO: 25, the amino acid sequence of HCDR2 is set forth in SEQ ID NO: 26, and the amino acid sequence of HCDR3 is set forth in SEQ ID NO: 27, or
the amino acid sequence of HCDR1 is set forth in SEQ ID NO: 31, the amino acid sequence of HCDR2 is set forth in SEQ ID NO: 32, and the amino acid sequence of HCDR3 is set forth in SEQ ID NO: 33;
and
for the light chain variable region of the antibody,
the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6,
the amino acid sequence of LCDR1 is set forth in SEQ ID NO: 10, the amino acid sequence of LCDR2 is set forth in SEQ ID NO: 11, and the amino acid sequence of LCDR3 is set forth in SEQ ID NO: 12,
the amino acid sequence of LCDR1 is set forth in SEQ ID NO: 16, the amino acid sequence of LCDR2 is set forth in SEQ ID NO: 17, and the amino acid sequence of LCDR3 is set forth in SEQ ID NO: 18,
the amino acid sequence of LCDR1 is set forth in SEQ ID NO: 22, the amino acid sequence of LCDR2 is set forth in SEQ ID NO: 23, and the amino acid sequence of LCDR3 is set forth in SEQ ID NO: 24,
the amino acid sequence of LCDR1 is set forth in SEQ ID NO: 28, the amino acid sequence of LCDR2 is set forth in SEQ ID NO: 29, and the amino acid sequence of LCDR3 is set forth in SEQ ID NO: 30, or
the amino acid sequence of LCDR1 is set forth in SEQ ID NO: 34, the amino acid sequence of LCDR2 is set forth in SEQ ID NO: 35, and the amino acid sequence of LCDR3 is set forth in SEQ ID NO: 36.

3. The anti-DKK1 antibody or the antigen-binding fragment thereof according to any one of claims 1 to 2, wherein,
the amino acid sequence of HCDR1 is set forth in SEQ ID NO: 1, the amino acid sequence of HCDR2 is set forth in SEQ ID NO: 2, the amino acid sequence of HCDR3 is set forth in SEQ ID NO: 3, the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6; the amino acid sequence of HCDR1 is set forth in SEQ ID NO: 1, the amino acid sequence of HCDR2 is set forth in SEQ ID NO: 2, the amino acid sequence of HCDR3 is set forth in SEQ ID NO: 78, the amino acid sequence of LCDR1 is set forth in SEQ ID NO: 4, the amino acid sequence of LCDR2 is set forth in SEQ ID NO: 5, and the amino acid sequence of LCDR3 is set forth in SEQ ID NO: 6; the amino acid sequence of HCDR1 is set forth in SEQ ID NO: 7, the amino acid sequence of HCDR2 is set forth in SEQ ID NO: 8, the amino acid sequence of HCDR3 is set forth in SEQ ID NO: 9, the amino acid sequence of LCDR1 is set forth in SEQ ID NO: 10, the amino acid sequence of LCDR2 is set forth in SEQ ID NO: 11, and the amino acid sequence of LCDR3 is set forth in SEQ ID NO: 12;
the amino acid sequence of HCDR1 is set forth in SEQ ID NO: 13, the amino acid sequence of HCDR2 is set forth in SEQ ID NO: 14, the amino acid sequence of HCDR3 is set forth in SEQ ID NO: 15, the amino acid sequence of LCDR1 is set forth in SEQ ID NO: 16, the amino acid sequence of LCDR2 is set forth in SEQ ID NO: 17, and the amino acid sequence of LCDR3 is set forth in SEQ ID NO: 18;
the amino acid sequence of HCDR1 is set forth in SEQ ID NO: 19, the amino acid sequence of HCDR2 is set forth in SEQ ID NO: 20, the amino acid sequence of HCDR3 is set forth in SEQ ID NO: 21, the amino acid sequence of LCDR1 is set forth in SEQ ID NO: 22, the amino acid sequence of LCDR2 is set forth in SEQ ID NO: 23, and the amino acid sequence of LCDR3 is set forth in SEQ ID NO: 24;
the amino acid sequence of HCDR1 is set forth in SEQ ID NO: 25, the amino acid sequence of HCDR2 is set forth in SEQ ID NO: 26, the amino acid sequence of HCDR3 is set forth in SEQ ID NO: 27, the amino acid sequence of LCDR1 is set forth in SEQ ID NO: 28, the amino acid sequence of LCDR2 is set forth in SEQ ID NO: 29, and the amino acid sequence of LCDR3 is set forth in SEQ ID NO: 30;
or
the amino acid sequence of HCDR1 is set forth in SEQ ID NO: 31, the amino acid sequence of HCDR2 is set forth in SEQ ID NO: 32, the amino acid sequence of HCDR3 is set forth in SEQ ID NO: 33, the amino acid sequence of LCDR1 is set forth in SEQ ID NO: 34, the amino acid sequence of LCDR2 is set forth in SEQ ID NO: 35, and the amino acid sequence of LCDR3 is set forth in SEQ ID NO: 36.

4. The anti-DKK1 antibody or the antigen-binding fragment thereof according to any one of claims 1 to 3, wherein,
the amino acid sequence of the heavy chain variable region of the antibody is selected from SEQ ID NO: 37, SEQ ID NO: 39, SEQ ID NO: 41, SEQ ID NO: 43, SEQ ID NO: 45, SEQ ID NO: 47, SEQ ID NOs: 49-53, SEQ ID NOs: 58-62, and SEQ ID NOs: 68-73; and
the amino acid sequence of the light chain variable region of the antibody is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs:74-77.

5. The anti-DKK1 antibody or the antigen-binding fragment thereof according to any one of claims 1 to 4, wherein,
the amino acid sequence of the heavy chain variable region of the antibody is set forth in SEQ ID NO: 37, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs:54-57, SEQ ID NOs: 63-67, and SEQ ID NOs: 74-77;
the amino acid sequence of the heavy chain variable region of the antibody is set forth in SEQ ID NO: 39, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 41, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 43, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 45, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 47, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 49, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 50, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 51, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 52, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 53, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 58, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 59, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 60, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 61, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 62, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 68, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 69, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 70, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 71, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
the amino acid sequence of the heavy chain variable region of the antibody is set forth in SEQ ID NO: 72, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77;
or
the amino acid sequence of the heavy chain variable region of the antibody is set forth in SEQ ID NO: 73, and the amino acid sequence of the light chain variable region is selected from SEQ ID NO: 38, SEQ ID NO: 40, SEQ ID NO: 42, SEQ ID NO: 44, SEQ ID NO: 46, SEQ ID NO: 48, SEQ ID NOs: 54-57, SEQ ID NOs: 63-67, and SEQ ID NOs: 74-77.

6. The anti-DKK1 antibody or the antigen-binding fragment thereof according to any one of claims 1 to 5, wherein,
the amino acid sequence of a heavy chain of the antibody is selected from SEQ ID NOs: 79-83, SEQ ID NOs: 88-92, and SEQ ID NOs: 98-103; and
the amino acid sequence of a light chain of the antibody is selected from SEQ ID NOs: 84-87, SEQ ID NOs: 93-97, and SEQ ID NOs: 104-107.

7. The anti-DKK1 antibody or the antigen-binding fragment thereof according to any one of claims 1 to 6, wherein,
the amino acid sequence of the heavy chain of the antibody is set forth in SEQ ID NO: 79, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 80, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 81, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 82, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 83, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 88, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 89, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 90, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 91, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 92, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 98, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 99, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 100, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 101, and the amino acid sequence of the light chain is selected from SEQ ID NOs: 84-87, SEQ ID NOs: 93-97, and SEQ ID NOs: 104-107;
the amino acid sequence of the heavy chain of the antibody is set forth in SEQ ID NO: 102, and the amino acid sequence of the light chain is selected from SEQ ID NOs: 84-87, SEQ ID NOs: 93-97, and SEQ ID NOs: 104-107;
or
the amino acid sequence of the heavy chain of the antibody is set forth in SEQ ID NO: 103, and the amino acid sequence of the light chain is selected from SEQ ID NOs: 84-87, SEQ ID NOs: 93-97, and SEQ ID NOs: 104-107.

8. The anti-DKK1 antibody or the antigen-binding fragment thereof according to any one of claims 1 to 7, wherein the anti-DKK1 antibody or the antigen-binding fragment thereof is selected from a Fab, a Fab', an F(ab')2, an Fd, an Fv, a dAb, a complementarity determining region fragment, a single-chain antibody, a humanized antibody, a chimeric antibody, or a diabody.

9. An anti-DKK1 antibody or an antigen-binding fragment thereof having the following characteristics:
(a) binding to an epitope of a human DKK1 protein that is identical to, completely overlaps with, or partially overlaps with an epitope to which the antibody or the antigen-binding fragment according to any one of claims 1 to 8 binds; or
(b) competing for binding to an epitope of a human DKK1 protein with the antibody or the antigen-binding fragment according to any one of claims 1 to 8.

10. The anti-DKK1 antibody or the antigen-binding fragment thereof according to any one of claims 1 to 9, wherein,
the antibody comprises a non-CDR region, and the non-CDR region is from a species except for murine, such as from a human antibody.

11. The anti-DKK1 antibody or the antigen-binding fragment thereof according to any one of claims 1 to 10, wherein a heavy chain constant region of the antibody is Ig gamma-1 chain C region (e.g., NCBI ACCESSION: P01857) or Ig gamma-4 chain C region (e.g., NCBI ACCESSION: P01861.1), and a light chain constant region is Ig kappa chain C region (e.g., NCBI ACCESSION: P01834); preferably, the amino acid sequence of the heavy chain constant region of the antibody is set forth in SEQ ID NO: 112, and the amino acid sequence of the light chain constant region of the antibody is set forth in SEQ ID NO: 113.

12. An isolated nucleic acid molecule encoding the anti-DKK1 antibody or the antigen-binding fragment thereof according to any one of claims 1 to 11.

13. A recombinant vector, comprising the isolated nucleic acid molecule according to claim 12.

14. A host cell, comprising the isolated nucleic acid molecule according to claim 12 or the recombinant vector according to claim 13.

15. A conjugate, comprising an antibody and a conjugated moiety, wherein the antibody is the anti-DKK1 antibody or the antigen-binding fragment thereof according to any one of claims 1 to 11, and the conjugated moiety is a detectable label; preferably, the conjugated moiety is a cytotoxin, an immunoregulator, a radioisotope, a fluorescent substance, a luminescent substance, a colored substance, or an enzyme.

16. A kit, comprising the anti-DKK1 antibody or the antigen-binding fragment thereof according to any one of claims 1 to 11 or the conjugate according to claim 15, wherein,
preferably, the kit further comprises a second antibody that specifically recognizes the antibody; optionally, the second antibody further comprises a detectable label, such as a cytotoxin, an immunoregulator, a radioisotope, a fluorescent substance, a luminescent substance, a colored substance, or an enzyme.

17. A bispecific antibody, comprising a first functional protein domain and a second functional protein domain, wherein,
the first functional protein domain targets DKK1, and
the second functional protein domain targets a target different from DKK1, wherein the first functional protein domain is the antibody or the antigen-binding fragment according to any one of claims 1 to 11.

18. The bispecific antibody according to claim 17, wherein the first functional protein domain and the second functional protein domain are linked directly or via a linker moiety.

19. The bispecific antibody according to any one of claims 17 to 18, wherein the number of the first functional protein domain and that of the second functional protein domain are independently 1, 2, or more than 2.

20. The bispecific antibody according to any one of claims 17 to 19, wherein the single-chain antibodies are linked to the C-termini of two heavy chains of an immunoglobulin-form antibody, respectively.

21. A pharmaceutical composition, comprising the anti-DKK1 antibody or the antigen-binding fragment thereof according to any one of claims 1 to 11, or the conjugate according to claim 15, or the bispecific antibody according to any one of claims 17 to 20, wherein,
optionally, the pharmaceutical composition further comprises one or more other anti-tumor agents; preferably, the other anti-tumor agent is a chemotherapeutic agent or an antibody; preferably, the chemotherapeutic agent is a tubulin inhibitor; preferably, the antibody drug is an immune checkpoint inhibitor; preferably, the chemotherapeutic agent is paclitaxel;
optionally, the pharmaceutical composition further comprises one or more pharmaceutically acceptable auxiliary materials.

22. A combination product, comprising a first product and a second product in separate packages, wherein,
the first product comprises the anti-DKK1 antibody or the antigen-binding fragment thereof according to any one of claims 1 to 11, or the conjugate according to claim 15, or the bispecific antibody according to any one of claims 17 to 20;
the second product comprises one or more other anti-tumor agents; preferably, the other anti-tumor agent is a chemotherapeutic agent or an antibody; preferably, the chemotherapeutic agent is a tubulin inhibitor; preferably, the antibody drug is an immune checkpoint inhibitor; preferably, the chemotherapeutic agent is paclitaxel;
preferably, the first product and the second product further independently comprise one or more pharmaceutically acceptable auxiliary materials;
preferably, the combination product further comprises a product instruction.

23. Use of the antibody or the antigen-binding fragment thereof according to any one of claims 1 to 11, the conjugate according to claim 15, the bispecific antibody according to any one of claims 17 to 20, or the pharmaceutical composition according to claim 21 in preparing a medicament for treating and/or preventing a DKK1-mediated disease, wherein preferably, the DKK1-mediated disease is a tumor.

24. The antibody or the antigen-binding fragment thereof according to any one of claims 1 to 11, the conjugate according to claim 15, the bispecific antibody according to any one of claims 17 to 20, or the pharmaceutical composition according to claim 21 for use in treating and/or preventing a DKK1-mediated disease, wherein preferably, the DKK1-mediated disease is a tumor.

25. A method for treating or preventing a DKK1-mediated disease, comprising the step of administering to a subject in need thereof an effective amount of the antibody or the antigen-binding fragment thereof according to any one of claims 1 to 11, the conjugate according to claim 15, the bispecific antibody according to any one of claims 17 to 20, or the pharmaceutical composition according to claim 21, wherein preferably, the DKK1-mediated disease is a tumor.

26. The method according to claim 25, further comprising the step of coadministering to the subject one or more therapies comprising surgical treatment and/or radiation therapy and/or administration of one or more other anti-tumor agents, wherein
preferably, the other anti-tumor agent is a chemotherapeutic agent or an antibody;
preferably, the chemotherapeutic agent is a tubulin inhibitor; preferably, the antibody drug is an immune checkpoint inhibitor; preferably, the chemotherapeutic agent is paclitaxel.
